# EUROPEAN PATENT SPECIFICATION

(11) **EP 0 623 101 B1**
(45) Date of publication and mention of the grant of the patent: **29.04.1998**
(21) Application number: 93901343.9
(22) Date of filing: 16.12.1992
(51) Int. Cl.: C07C 2/84, C07C 5/48, B01J 23/10, B01J 23/14

(54) **HYDROCARBON CONVERSION**
UMWANDLUNG VON KOHLENWASSERSTOFFEN
CONVERSION D'HYDROCARBURES

(30) Priority: 31.12.1991 US 815244
(43) Date of publication of application: 09.11.1994
(73) Proprietor: AMOCO CORPORATION, Chicago, IL 60680-0703 (US)
(72) Inventor: KAMINSKY, Mark, P., Winfield, IL 60190 (US); KLEEFISCH, Mark, S., Naperville, IL 60540 (US); HUFF, George, A., Jr., Naperville, IL 60563 (US); WASCHECHECK, Don, M., Naperville, IL 60565 (US); BARR, Mark, K., Wheaton, IL 60187 (US); SHUM, Victor, K., Naperville, IL 60563 (US)
(74) Representative: Ritter, Stephen David
(86) International application number: PCT/US92/10777
(87) International publication number: WO 93/13037

(56) References cited:
- WO-A-90/02109
- DE-A- 4 103 431
- US-A- 2 549 844
- US-A- 4 656 155

## Description

### BACKGROUND OF THE INVENTION

This invention relates generally to the conversion of hydrocarbons and, more specifically, to contact material compositions and oxidative conversion processes using such compositions.

As the uncertain nature of the limited supplies of and access to crude oil has become increasingly apparent, alternative sources of hydrocarbons and fuels have been sought out and explored. The conversion of low molecular weight alkanes (lower alkanes) to higher molecular weight hydrocarbons has received increasing consideration as such low molecular weight alkanes may be generally available from more readily secured and reliable sources. Natural gas, partially as a result of its comparative abundance, has received a large measure of the attention that has focused on sources of low molecular weight alkanes. Large deposits of natural gas, mainly composed of methane, are found in many locations throughout the world. In addition, low molecular weight alkanes are generally present in coal deposits and may be formed during numerous mining operations, in various petroleum processes, and in the above- or below-ground gasification or liquefaction of coal, tar sands, oil shale and biomass, for example.

Today, much of the readily accessible natural gas generally has a high valued use as a fuel whether in residential, commercial or in industrial applications. Additional natural gas resources, however, are prevalent in many remote regions of the world, such as remote areas of Western Canada, Africa, Australia, U.S.S.R. and Asia. Commonly, natural gas from these remote resources is referred to as "remote natural gas" or, more briefly, "remote gas."

In many such remote regions, the widespread, direct use of the natural gas as a fuel is generally not currently profitable. Further, the relative inaccessibility of gas from such resources is a major obstacle to the more effective and extensive use of remote gas as the transportation of the gas to distant markets wherein the natural gas could find direct use as a fuel is typically economically unattractive.

Of course, while the primary current use of natural gas is as a fuel, natural gas may alternatively be used as a feedstock for chemical manufacture. In fact, natural gas is a primary chemical feedstock for the manufacture of numerous chemicals, such as methanol, ammonia, acetic acid, acetic anhydride, formic acid, and formaldehyde, for example. However, the markets for such chemicals are fairly limited in size. Consequently, methods for converting low molecular weight alkanes, such as those present in remote natural gas, to higher molecular weight hydrocarbons, preferably, to more easily transportable liquid fuels for which the world market is relatively large and/or elastic, are desired and a number of such methods have been proposed or reported.

Conversion of natural gas to liquid products is a promising solution to the problem of more effectively and efficiently utilizing low molecular weight hydrocarbons from remote areas and constitutes a special challenge to the petrochemical and energy industries. The dominant technology currently employed for the utilization of remote natural gas involves conversion of the natural gas to a liquid form via the formation of synthesis gas, i.e., a process intermediary composed of a mixture of hydrogen and carbon monoxide also commonly referred to as "syngas." In syngas processing, methane, the predominant component of natural gas, although typically difficult to activate, is reacted with oxygen or oxygen-containing compounds such as water or carbon dioxide to produce syngas which in turn is then converted to desired products.

Syngas processing, however, is relatively costly as the production of syngas and the subsequent conversion of the syngas are typically very capital intensive processing schemes. Further, while some of the products to which syngas can be converted, such as methanol, mixed alcohols, acetic acid, etc., contain oxygen and are thus logical products for production via syngas processing, hydrocarbon products such as gasoline and diesel fuel typically do not contain oxygen and consequently the production of such materials via syngas processing requires the additional processing step of oxygen removal. Consequently, when such products are produced via syngas processing, the addition and later removal of oxygen ultimately increases the cost of production.

When hydrocarbon products such as gasoline and diesel fuel are sought, the syngas mixture can be converted to syncrude, such as with Fischer-Tropsch technology, and then upgraded to the desired transportation fuels using typical refining methods. Alternatively, syngas can be converted to liquid oxygenates which can be blended with conventional transportation fuels to form materials such as gasohol, used as an alternative fuel or converted to conventional transportation fuels by catalysts such as certain zeolites.

Because syngas processing typically requires high capital investment, with syngas typically being produced in energy intensive ways such as by steam reforming where fuel is burned to supply the heat of reforming, and represents an indirect means of higher hydrocarbon production (i.e., such processing involves the formation and subsequent reaction of the syngas intermediaries), other means for converting lower alkanes directly to higher hydrocarbons have been sought.

Oxidative coupling has been recognized as a promising approach to the problem of conversion of lower alkanes to higher molecular weight hydrocarbons. The mechanism of action of oxidative coupling processing, however, has not been clearly identified or defined and is not clearly understood. In such oxidative coupling processing, a low molecular weight alkane or a mixture containing low molecular weight alkanes, such as methane, is contacted with a solid material referred to by various terms including catalyst, promoter, oxidative synthesizing agent, activator or contact material. In such processing, the methane is contacted with such a "contact material" and, depending on the composition of the contact material, in the presence or absence of free oxygen gas, is directly converted to ethane, ethylene, higher hydrocarbons and water. Carbon dioxide, the formation of which is highly favored thermodynamically, is an undesired product, however, as the formation of carbon dioxide results in both oxygen and carbon being consumed without production of the desired higher value C₂₊ hydrocarbons.

Catalytic mixtures containing reducible metal oxides are highly active and many are 100% selective for producing CO₂, that is, they are combustion catalysts. In order to obtain desired selectivity for hydrocarbon formation, Group IA metals, particularly lithium and sodium, have been used in such catalytic mixtures. Under the conditions used for oxidative coupling, however, migration and loss of the alkali metal normally occurs. In order to avoid complete combustion most methods for oxidative conversion have been carried out in the absence of an oxygen-containing gas, relying on the oxygen theoretically being supplied by the catalyst.

Nevertheless, in most cases involving oxidative coupling processing of methane, carbon monoxide and hydrogen are coproduced in addition to desired C₂₊ hydrocarbons. If desired, such coproduced hydrogen can be used alone, in part or in its entirety, or supplemented with hydrogen from another source to effect conversion of carbon oxides to produce methane. Such produced methane can, in turn, be recycled for desired oxidative coupling processing. Alternatively, the hydrogen can be used to effect conversion of carbon monoxide to carbon-containing oxygenates such as methanol or mixed alcohols (e.g., a mixture of one or more alcohols such as methanol, ethanol, propanols and butanols) or higher hydrocarbons such as a mixture of paraffins and olefins typically produced in the process commonly known as Fischer-Tropsch synthesis. Alternatively or in addition, such coproduced carbon monoxide and hydrogen can, if desired, be combined with olefins, such as those produced during the oxidative coupling processing, to produce various oxygenates, such as propanol, for example. As described above, however, the production of materials such as oxygenates from carbon monoxide and hydrogen (i.e., synthesis gas) is not a direct approach for the utilization of natural gas, as such processing still involves the use of the syngas intermediaries.

Furthermore, the processing of coproduced hydrogen and carbon monoxide typically increases the cost of any such processing scheme. Thus, the need for active oxidative coupling contact materials which have relatively high selectivities for desired higher hydrocarbons and which contact materials are stable and have long life (i.e., maintain relatively high levels of activity and selectivity to higher hydrocarbons over extended periods of use without the need for regeneration or replacement).

Many patents describe processes for converting methane to heavier hydrocarbons in the presence of reducible metal oxide catalysts. During such processing, the reducible metal oxide "catalyst" typically is reduced and thus most of these patents require or imply the need for a separate stage to reoxidize the catalyst.

For example, U.S. Pat. No. 4,444,984 discloses a method for synthesizing hydrocarbons wherein methane is contacted with a reducible oxide of tin at an elevated temperature. Such contact results in the tin oxide being reduced. The reduced composition is then oxidized with molecular oxygen to regenerate a reducible oxide of tin.

U.S. Pat. No. 4,495,374 discloses the use of a reducible metal oxide promoted by an alkaline earth metal in such a method of methane conversion. During such processing, the reducible metal oxide of the promoted oxidative synthesizing agent is reduced. The reduced synthesizing agent can then be removed to a separate zone wherein it is contacted with an oxygen-containing gas to regenerate the promoted oxidative synthesizing agent.

Examples of other such patents include; U.S. Pat. No. 4,523,049, which shows a reducible oxide catalyst promoted by an alkali or alkaline earth meta, and requires the presence of oxygen during the oxidative coupling reaction; U.S. Pat. No. 4,656,155, which specifies a reducible metal oxide in combination with an oxide of zirconium, an oxide of yttrium and, optionally, an alkali metal; U.S. Pat. No. 4,450,310, which is directed to coupling promoted by alkaline earth metal oxides in the total absence of molecular oxygen; and U.S. Pat. No. 4,482,644, which teaches a barium-containing oxygen-deficient catalyst with a perovskite structure.

Several patents describe catalysts for higher hydrocarbon synthesis which can include a Group IIA metal; scandium, yttrium or lanthanum; and/or other metal oxides.

DE-A-4 103 431 contains a generic disclose of a process for converting methane into higher hydrocarbons using an oxide catalyst represented by the empirical formula:$\text{Aa Bb Cc Ox ,}$ where:
A is a single element selected from Ge, Si, Sn, Ti, and Zr;
B is a single element selected from La, Sc, and Y;
C is a single alkali- or alkaline-earth metal;
a, b, c, are in the range of 0.01-10; and x is the total valence of the different elements A, B and C.

While this formula includes numerous permutations and combinations of metal components with oxygen, all other elements are precluded anionic species.

Commonly assigned U.S. Pat. No. 4,931,311 (WO-A-9 02 109), discloses a catalyst composition comprising a mixed oxide of:
a) a Group IIIB metal selected from the group consisting of yttrium, scandium and lanthanum;
b) a Group IIA metal selected from the group consisting of barium, calcium and strontium; and
c) a Group IVA metal selected from the group consisting of tin, lead and germanium, with the Group IIIB, Group IIA and Group IVA metals in an approximate mole ratio of 1:0.5-3:2-4, respectively.

U.S. Patent No. 4,780,449 discloses a catalyst including metal oxides of a Group IIA metal, a Group IIIA metal, a lanthanide series metal excluding Ce, or mixtures thereof. The patent lists as optional promoter materials metal oxides of a metal of Groups IA, IIA, IIIA, IVB, VB, IB, the lanthanide series, or mixtures thereof.

Catalysts which contain metal oxides which are reduced under the reaction conditions of use are typically physically and/or chemically relatively unstable under the reaction conditions of use. That is, such catalysts generally do not maintain needed or desired physical and/or chemical characteristics for extended periods of time (e.g., such characteristics as reactivity and physical form are typically not maintained for more than a few minutes) without regeneration, reformation or other remedial procedures.

Also, as the reducible metal oxides of such materials typically undergo chemical reduction with use, the activity of the materials for producing desired products, such as C₂₊ hydrocarbons in the oxidative coupling processing of methane, for example, wersen.

For example, with contact materials containing reducible metal oxides, the problem of over-reduction is typically associated with the reduction of the metal oxide to the metal. Often, the selectivity of the contact material changes dramatically when the material has been over-reduced, leading to a material which results in combustion reactions or which results in the formation of mixtures of carbon oxides with water and hydrogen when the material is used in the oxidative coupling of lower alkanes such as methane, for example. Some reducible metal oxide-containing contact materials (e.g., manganese oxide) at temperatures in the range of about 800° to about 1,000°C and in the absence of oxygen, once over-reduced are very difficult to reoxidize and a permanent or near permanent alteration in the characteristics of the material occurs. In some cases, the reduced metal can react with other materials in the composition to form a new phase which is difficult to reoxidize and the contact material is permanently damaged by over-reduction. Such alterations can, for example, result in a loss in selectivity to C₂₊ hydrocarbons when the material is used in the oxidative coupling of methane.

Furthermore, contact materials containing metal oxides which are reducible under the reaction conditions of use can, during such use, experience physical deterioration, e.g., breaking apart. Such physical deterioration results, at least in part, from changes in the material during oxidation and reduction. Frequently, the material in its various oxidation states has very different densities, e.g., the material contracts and swells as it is reduced and oxidized. The smaller particles or powders, frequently referred to as "fines," resulting when the material undergoes physical degradation results in pressure drop buildups (in fixed bed operation) and leads to loss of contact material (in fluid bed operation).

In fluid bed operation, fines are frequently carried out with the vapors from the reactor. Additionally, the fines are generally not easily separated from the product gases in common separating devices such as cyclones. Thus, costly separation techniques are required to effect separation of the fines from the product gases. The loss of contact material in the form of fines also necessitates the addition of more contact material to the process to replace that which has been lost and thereby increases the cost of such processing.

The search for a stable, long-lived contact material having high activity and selectivity in the oxidative conversion processing of hydrocarbons has continued.

According to one aspect of the invention there is provided a method for converting lower alkanes to a product composition comprising a higher molecular weight hydrocarbon, comprising contacting a feed composition comprising at least one lower alkane material with an oxidative coupling contact material consisting of an intimately mixed, mixed oxide of:
a) at least one cationic species of a naturally occurring Group IIIB element selected from the group consisting of yttrium, lanthanum, neodymium, samarium and ytterbium;
b) at least one cationic species of a Group IIA metal selected from the group consisting of magnesium, calcium, strontium and barium, where the Group IIIB element cationic species and the Group IIA metal cationic species are present in an approximate molar ratio of about 1 Group IIIB element cationic species to about 0.5 to about 3 of the Group IIA metal cationic species; and
c) at least one additional metal cationic species selected from zirconium, hafnium, germanium, gallium and aluminum and wherein:
   i) when selected from zirconium and hafnium, said additional metal cationic species is present in an amount in a range of about 10 to 55 weight percent of the oxidative coupling contact material;
   ii) when selected from germanium and gallium, said additional metal cationic species is present in a molar ratio of about 1 mole of Group IIIB element cationic species to about 0.5 - 4 moles of the additional metal cationic species, and wherein the oxidative coupling contact material contains a halide, present in an amount in a range of about 5 to 20 weight percent of the oxidative coupling contact material; and
   iii) when selected from aluminum, said additional metal cationic species is present in an amount in a range of about 2 to 20 weight percent of the oxidative coupling contact material;
with the contacting being at oxidative coupling reaction conditions and in the presence of oxygen.

The invention further provides an oxidative coupling contact material consisting of an intimately mixed, mixed oxide of:
a) at least one cationic species of a Group IIIB element selected from the group consisting of yttrium, lanthanum, neodymium, samarium and ytterbium;
b) at least one cationic species of a Group IIA metal selected from the group consisting of strontium and barium, where the Group IIIB element cationic species and the Group IIA metal cationic species are present in an approximate molar ratio of about 1 Group IIIB element cationic species to about 0.5 to about 3 of the Group IIA metal cationic species; and
c) at least one additional metal cationic species selected from zirconium, hafnium, germanium, gallium and aluminum and wherein:
   i) when selected from zirconium and hafnium, said additional metal cationic species is present in an amount in a range of about 10 to 55 weight percent of the oxidative coupling contact material;
   ii) when selected from germanium and gallium, said additional metal cationic species is present in a molar ratio of about 1 mole of Group IIIB element cationic species to about 0.5 - 4 moles of the additional metal cationic species, and wherein the oxidative coupling contact material contains a halide, present in an amount in a range of about 5 to 20 weight percent of the oxidative coupling contact material; and
   iii) when selected from aluminum, said additional metal cationic species is present in an amount in a range of about 2 to 20 weight percent of the oxidative coupling contact material.

The stable contact material compositions of the invention, by being able to maintain needed or desired physical and/or chemical characteristics for extended periods, permit the use thereof in processing without necessitating troublesome and/or costly remedial procedures.

In another aspect of this invention, preferable Group IIIB elements include yttrium, lanthanum, neodymium, samarium and ytterbium. The most preferable Group IIIB element is yttrium.

In one aspect of this invention, the cationic species incorporated into compositions of this invention is aluminum.

In another aspect, the preferable cationic species incorporated into compositions of this invention is zirconium or hafnium, and the most preferable is zirconium.

In one aspect of this invention, the cationic species incorporated into compositions of this invention is germanium or gallium.

In another aspect of the invention, compositions containing an germanium or gallium cationic species contain a halogen.

The invention also comprehends methods for the conversion of lower alkanes to higher molecular weight hydrocarbons. In such methods, a feed composition including at least one lower alkane species is contacted with the specified contact material composition. Such contacting is done in the presence of oxygen and at oxidative coupling reaction conditions.

The invention also comprehends methods for the oxidative dehydrogenation of dehydrogenatable hydrocarbons. In such methods, oxygen and a gas containing a dehydrogenatable hydrocarbon are contacted with the specified contact material composition at oxidative dehydrogenation reaction conditions to produce an effluent containing dehydrogenated hydrocarbons.

The invention also comprehends methods for the oxidative cracking of crackable hydrocarbons. In such methods, oxygen and a gas containing a crackable hydrocarbon are contacted with the specified contact material composition at oxidative cracking reaction conditions to produce an effluent containing cracked hydrocarbons.

### BRIEF DESCRIPTION OF THE DRAWINGS

Figs. 1 and 2 are graphical depictions of O₂ conversion and C₂₊ selectivity, respectively, versus reaction temperature for conversion of methane to higher molecular weight hydrocarbons according to typical embodiments of the invention.

Figs. 3, 4 and 5 are graphical depictions of O₂ conversion and C₂₊ selectivity versus time onstream for conversion of methane to higher molecular weight hydrocarbons according to typical embodiments of the invention.

Fig. 6 is a graphical depiction of CH₄ conversion, O₂ conversion and C₂₊ selectivity versus feed flow rate both with and without N₂ dilution for conversion of methane to higher molecular weight hydrocarbons according to typical embodiments of the invention.

Figs. 7 and 8 are graphical depictions of C₂₊ selectivity and O₂ conversion, respectively, versus time on stream for conversion of methane to higher molecular weight hydrocarbons according to a typical embodiment of the invention as well as a process utilizing comparative contact materials.

### DETAILED DESCRIPTION OF THE INVENTION

According to the invention, an oxidative coupling contact material and methods for hydrocarbon conversion are provided. The invention contemplates an oxidative coupling contact material composition which is substantially free of catalytically effective reducible metal oxide and methods of hydrocarbon conversion utilizing such contact material compositions including:
a) methods for alkane conversion generally applicable to alkanes containing from 1 to 3 carbon atoms to form higher molecular weight hydrocarbons,
b) methods for dehydrogenation of dehydrogenatable hydrocarbons; and
c) methods for the cracking of crackable hydrocarbons.

In one preferred embodiment of the invention, methane, illustrative of a lower molecular weight alkane feedstock useful in the practice of the invention, is mixed with air, as a source of oxygen, and the resulting mixture is contacted with a suitable oxidative coupling contact material, as described below, for the oxidative coupling of the aforesaid alkane. Thus, the invention will be described herein with reference to conversion wherein the lower alkanes converted to higher molecular weight hydrocarbons comprise methane. It is to be understood, however, that feedstocks typically useful in the practice of the invention will include lower alkanes such as methane, ethane or propane (i.e., C₁-C₃ alkanes) either alone, separately or in mixtures with each other, with or without the presence of other materials, such as inert gases, e.g., N₂ or minor amounts of other hydrocarbon materials, for example. Natural gas is an example of a feedstock for use in the practice of at least some aspects of the invention. It being understood that natural gas, while containing predominantly methane, can and typically does contain at least minor amounts of the other above-identified lower alkanes as well as other materials such as nitrogen gas and carbon dioxide, for example. Also, It is to be understood, that the method can be utilized with higher alkane feedstocks. As a result of competing reaction kinetics, however, such use can result in a reduction in the amount of higher molecular weight hydrocarbons formed thereby.

It is also to be understood that in the hydrocarbon conversion methods of the invention, sources or forms of oxygen-containing gas other than air can be used or preferred. Thus, the oxygen-containing gas for use in the conversion methods of the invention can vary in molecular oxygen content from oxygen-depleted air, to air, to oxygen gas itself, for example. Air or enriched air can be a preferred source of molecular oxygen.

Such oxidative coupling processing of methane, when air is used as a source of oxygen, typically results in a gaseous mixture comprising ethane and ethylene, illustrative of saturated and unsaturated aliphatic hydrocarbon products having higher molecular weights than the feedstock alkanes from which they were formed, and possibly some traces of aromatics or higher hydrocarbons which may form in the reactor, such as at high operating temperatures, for example, at temperatures greater than 750°C, as well as carbon monoxide, carbon dioxide, nitrogen, water, any remaining unreacted feedstock alkane and oxygen. It being understood that conventional catalytic processing schemes, such as refining hydrotreatment, are typically conducted at operating temperatures of only about 400°C to 450°C.

Such a reaction product mixture may illustratively be used as chemical feedstock or be further reacted, such as occurs during conversion, to form gasoline type hydrocarbon products. For example, the effluent with desired or required pretreatment, e.g., H₂O removal, and/or downstream treatment, e.g., N₂ removal, may be passed over a suitable aromatization/oligomerization catalyst (such as a crystalline borosilicate or aluminosilicate molecular sieve material or supported phosphoric acid) to produce desired gasoline-type hydrocarbon products. Other specific uses of the reactor effluent will be apparent to those skilled in the art.

As used herein, the term "reducible" is used to identify those oxides of metals which are reduced by contact with C₁-C₃ alkanes at temperatures within the range of about 500°C to about 1,000°C.

The terms "dehydrogenatable hydrocarbons" and "crackable hydrocarbons" include not only conventional hydrocarbons, which contain exclusively hydrogen and carbon, but also compounds, which in addition to hydrogen and carbon, contain oxygen as well, e.g., compounds such as alcohols (C₂₊ and C₃₊ alcohols, for example).

The term "catalytically effective" refers to the ability of the material in question to increase chemical reactivity for the formation of hydrocarbons in preference to carbon oxide (CO and CO₂) formation.

The terms "oxide" and "oxides" include the various oxygen-containing compositions including hydroxides, carbonates, peroxides, superoxides and mixtures thereof, for example.

The term "lower alkane" as used herein refers to C₁-C₃ alkanes.

The term "contact material" as used herein refers to a material which:
(a) when contacted with a lower alkane and oxygen at oxidative coupling reaction conditions results in the formation of hydrocarbons having a higher molecular weight than the original feed alkane, or
(b) when contacted with a higher hydrocarbon (e.g., butanes, pentanes, hexanes and mixtures thereof) at oxidative dehydrogenation or oxidative cracking reaction conditions leads to a dehydrogenation and/or molecular weight reduction, respectively, of the higher hydrocarbon, generally with the formation of dehydrogenated lower molecular weight hydrocarbons.

The term "cofeed" operation as used herein refers to that mode of conversion operation wherein the contact material is simultaneously contacted by the feed materials, e.g., feed hydrocarbon, and oxygen (such as in the form of an oxygen-containing gas). In such operation, the feed material and the oxygen can be mixed together before or during their contact with the contact material.

The term "redox" operation as used herein refers to that mode of conversion operation wherein the contact material is sequentially contacted by the feed materials, e.g.,feed hydrocarbon, followed by contact with oxygen (such as in the form of an oxygen-containing gas). In such operation, the feed material and oxygen are generally not mixed together to any substantial extent either before or during contact with the contact material. In some process designs, however, some such "carryover" or inadvertent mixing of the feed material and oxygen may occur.

The term "gasoline-type hydrocarbon products" as used herein refers to those hydrocarbons having a boiling point in the general range of C₄ hydrocarbons to about 450°F (232°C), inclusive.

The term "substantially free" as used herein to describe the contact material composition generally indicates that the contact material composition excludes amounts of the specified material(s) which materially affect the effectiveness of the contact material in the specified processing. While the affect of a specified material on the effectiveness of the contact material will, of course, be dependent on the material and processing involved, "substantially free" means that the contact material composition includes no more than nominal amounts of the specified materials, typically the composition contains an amount of no more than about 0.1 wt.%, more specifically the composition contains an amount of no more than about 100 ppm (0.01 wt.%) and more preferably the composition contains an amount of no more than about 50 ppm (0.005 wt.%) of the specified materials.

The term "intimately mixed" as used herein refers to mixing of the different contact material cationic species, either alone or in some compound form, on a molecular level. The term is descriptive of and refers to materials which when thin sectioned to about 90 nanometers or dispersed on a carbon film and scanned over a spot of no more than about 5 to 10 square microns, preferably a spot of no more than about 1 to 5 square microns and, more preferably, a spot of no more than about 0.1 to 1 square micron by way of Scanning Transmission Electron Microscopy with Energy Dispersive X-Ray Analysis (STEM-EDX) exhibits each of the three principal metal cationic species of the material in significant amounts (i.e., more than contaminant or impurity amounts).

In the above-described embodiment, methane and oxygen (as a part of air) are simultaneously contacted with the oxidative coupling contact material. Such operation is commonly referred to as "cofeed" operation and in such operation, oxygen, which may be needed for the coupling reaction to occur, is also fed to the reactor rather than exclusively being carried into the reactor via the lattice of the contact material, as may be typical of "redox" operation, as described above. Further, cofeed operation may minimize or eliminate the need for subsequent reoxidation of the contact material such as may be required to resupply lattice oxygen to contact materials such as those which typically contain reducible metal oxides as typically is required when such contact materials are utilized in a redox mode operating scheme.

Generally, a suitable feedstock for the method of this invention comprises at least one of methane, ethane and propane and preferably comprises mostly methane, e.g., at least about 75 percent methane, and more preferably may be methane as methane is typically the predominant hydrocarbon reserve component which is desired to be converted to a higher molecular weight hydrocarbon. Thus, a suitable feedstock for the method of this invention comprises natural gas, gases formed during mining operations and petroleum processes or in the above- or below-ground gasification or liquefaction of coal, tar sands, oil shale and biomass, for example.

The contacting of the hydrocarbon feedstock with the oxygen-containing gas and in the presence of the contact material generally is performed at oxidative coupling reaction conditions including temperature and pressure. Preferably, such contacting is performed at a temperature in the range of from about 600°C to about 1,000°C and, more preferably, in the range of from about 700°C to about 900°C. These temperature ranges have been found to be preferred as operation at temperatures below about 600°C may generally result in the contact material having relatively unfavorable product (e.g., C₂₊ hydrocarbons) selectivities while operation at higher temperatures, e.g., temperatures greater than about 900°C, can result in generally undesirable thermal reactions seriously competing with coupling reactions. The products resulting from such thermal reactions will typically be largely comprised of H₂, COₓ (where x = 1 or 2) and may also include coke, acetylene and aromatics such as benzene, for example. Such thermal reactions will typically overwhelm the desired coupling reactions when temperatures exceed about 1,000°C. It is to be understood, however, that at higher reaction temperatures at least trace quantities of aromatic compounds may also form.

The contacting of the hydrocarbon feedstock and oxygen with the contact material is preferably performed under a total absolute pressure in the range of from about 0.1 atmosphere to about 10 atmospheres, and more preferably in the range of from about 1 atmosphere to about 5 atmospheres, as operation at pressures exceeding this range typically results in reduced C₂₊ product selectivities while subatmospheric operation is believed to be economically unattractive as capital expenditures escalate rapidly for a system to be capable of handling the actual volumes of gas required for such a commercial operation.

The ratio of the partial pressure of the combined feedstock alkanes containing from 1 to 3 carbon atoms to the oxygen partial pressure at the entrance of the reactor in the contacting step is preferably in the range of from about 2:1 to about 40:1 and, more preferably, in the range of from about 2:1 to about 10:1, as operation at lower C₁-C₃ alkane to oxygen partial pressure ratios generally results in excessive carbon oxide formation, while operation at higher ratios may result in insufficient amounts of oxygen being present to obtain desired levels of conversion and consequently results in the remainder of greater amounts of unreacted hydrocarbon reactant. The combined partial pressures of the alkanes in the feedstock containing from 1 to 3 carbon atoms at the entrance to the first reactor (the contacting reactor) is preferably no more than about 10 atmospheres, and, more preferably, no more than about 4 atmospheres. The oxygen partial pressure at the entrance to the first reactor is preferably no more than about 4 atmospheres and, more preferably, no more than about 2 atmospheres. The oxygen partial pressure in the gaseous effluent from the reactor in the contacting step is preferably substantially 0.

In view of the highly active nature of the subject contact materials for the oxidative conversion of lower alkanes to a product composition containing higher molecular weight hydrocarbons, the contacting step is preferably performed at a space velocity of from about 1,000 to about 1,000,000 volumes of total feed gas at ambient conditions per volume of catalytic composition per hour and more preferably at a space velocity of about 50,000 to about 200,000 volumes of total feed gas per volume of catalytic composition per hour, as thermal reactions will generally predominate with operation at lower space velocities while oxygen conversion will generally be unsuitably incomplete with operation at higher space velocities.

The high activity of the subject contact materials combined with the release of large amounts of heat associated with the exothermic oxidative coupling reaction of lower alkanes, makes heat transfer and temperature control significant engineering challenges to commercial operation of the process. Reactors particularly suited for use in the practice of the invention need to allow for heat transfer and permit desired temperature control. Such reactors can include certain types of fluidized bed reactors wherein the contact material is finely divided as this promotes a more rapid heat transfer as well as tubular reactors wherein the contact material is directly applied to the reactor wall to promote heat transfer and to permit desired temperature control.

As indicated the present invention provides an intimately mixed contact material composition substantially free of a catalytically effective reducible metal oxide and containing at least three different cationic species.

It has been found that materials with an intimate mixture of at least one cationic species of a Group IIIB and at least one cation species of a Group IIA metal, selected from the group consisting of magnesium, calcium, strontium and barium, have improved performance characteristics, e.g., higher C₂₊ selectivities when used in the oxidative coupling processing of methane, as compared to the corresponding contact materials wherein the Group IIIB species and the Group IIA species are used alone or in which the species are present in a non-intimately mixed fashion.

It has been found that a cationic species of zirconium or hafnium in the intimate mixture results in a contact material with improved stability and improved physical properties such as improved hardness and attrition resistance, as is desired when fluidizable contact materials are sought, as compared to similar materials but which do not contain an intimately mixed zirconium or hafnium species.

Such compositions will preferably contain no more than about 75 weight percent zirconium or hafnium and, more preferably, will contain zirconium or hafnium in an amount in the range of about 10 to 55 weight percent of the contact material composition. (Such weight percents being on an elemental basis). Such relative amounts of zirconium and hafnium in the contact materials of the invention are preferred because they result in more active and selective contact materials, e.g., in the oxidative coupling of methane, such contact materials result in higher conversions and selectivities to C₂₊ hydrocarbons. In addition, more than nominal amounts of zirconium and/or hafnium, i.e., typically more than about 1 to 2 weight percent on an elemental basis, is believed required. It is theorized that zirconium and hafnium act to keep the Group IIA metal, e.g., barium, from volatizing out of the contact material. For example, relatively stable Ba-Zr and Ba-Hf oxide phases, respectively, are formed, and serve to deter or avoid such volatization.

Also, in such compositions, the cationic species of the Group IIIB element and the Group IIA metal are preferably present in an approximate molar or atomic ratio of about one Group IIIB element cationic species to about 0.001 to about 100 Group IIA metal cationic species and, more preferably, in a ratio of about 1 Group IIIB element cationic species to about 0.5 to about 3 Group IIA metal cationic species, and even more preferred, a ratio of about 1 Group IIIB element cationic species to about 1.5 to about 2.5 Group IIA metal cationic species.

Further, it has been found that a cationic species of aluminum in the intimate mixture results in a contact material with improved stability and improved physical properties such as improved hardness and attrition resistance, as is desired when fluidizable contact materials are sought, as compared to similar materials but which do not contain an intimately mixed aluminum species.

Such compositions will preferably contain about 2 to 20 weight percent aluminum and, more preferably, will contain aluminum in an amount in the range of 5-10 weight percent of the contact material composition (such weight percents being on an elemental basis). Such relative amounts of aluminum are generally preferred as such compositions will generally exhibit desired chemical and physical stability, as well as desired reactivity and selectivity. For example, and without wishing to be bound by any theory or mode of operation, it is believed that compositions containing only lesser relative amounts of aluminum do not result in sufficient chemical interaction to minimize or avoid the loss of other composition elements or sufficiently contribute to the hardness of the resulting composition, e.g., chemical and physical stability, respectively.

In addition, those compositions containing aluminum, typically present as the oxide, in higher relative amounts, can have an adverse effect on the reactivity and/or selectivity of the composition. For example, the higher relative amount of aluminum can dilute the relative amounts of other composition components to such an extent that the reactivity of the composition is reduced.

Another factor which limits the relative amount of aluminum in these compositions is the effect of incorporation of aluminum as the oxide on the surface area of the composition. Generally, when the surface area of the contact material becomes too great, e.g., greater than about 150 m²/g, more particularly greater than about 50 m²/g and even more particularly greater than about 20 m²/g, such as can occur when the alumina content of the material is too high, the selectivity of the composition is significantly and dramatically altered. For example, when used in oxidative coupling of lower alkanes, the use of a contact material composition with too large of a surface area generally leads to increased formation of carbon oxides, as opposed to the formation of additional desired higher hydrocarbons. Further, when used in oxidative dehydrogenation or oxidative cracking of higher hydrocarbons, the use of a contact material composition having too large a surface area can result in increased carbon deposition on the contact material.

Carbon deposition not only results in the loss of carbon from the feed by the formation of undesired products, e.g., coke, it can also result in a loss of activity for the contact material. For example, when using a fixed bed reactor, as the activity of the contact material decreases because of increasing carbon deposition, the process periodically must be interrupted and the contact material regenerated by the removal of some of the carbon formed thereon, such as by the oxidation of the carbon. Of course, such periodic regenerations of the process results in a loss of process productivity. Further, during the removal of the carbon such as by an exothermic process, the contact material can be damaged by reaching elevated temperatures in sections or areas during the regeneration. Such sections or areas of high temperature are commonly referred to as "hot spots." Hot spots can irreversibly damage the contact material such as through sintering and result in a loss in activity, selectivity or physical strength for the material. Alternatively, when the process uses a fluid bed of contact material and the material experiences a loss of activity as a result of carbon deposition, the material generally will be continuously removed from the reactor vessel to permit the removal of the carbon from the contact material in a separate vessel, e.g., a regenerator. If the amount of the carbon deposition on the contact material is excessive, heat balancing of the process can be difficult to achieve and control. Regardless of the type of reactor used, carbon deposition can also lead to a loss of physical strength in the contact material.

Also, in such aluminum-containing compositions, the cationic species of the Group IIIB element and the Group IIA metal are preferably present in an approximate molar or atomic ratio of about 1 to about 0.5-3 and, more preferably, in a ratio of about 1 to about 2.

In halogen-containing compositions of this invention which contain germanium or gallium, the halogen can be fluorine, chlorine, bromine or iodine. Chlorine is generally preferred as chlorine generally results in contact materials which in use, for example, in the oxidative conversion of methane or other lower hydrocarbons, particularly lower alkanes to higher hydrocarbons, results in improved performance, e.g., better selectivities for desired products, such as C₂₊ hydrocarbons in such conversion processing of methane, as compared to the other named halogen family members. In addition, chlorine tends not to volatize as readily from such contact materials as halogen family members such as bromine and iodine.

One theorized explanation for the higher C₂₊ selectivities realized in the oxidative conversion of methane when using such contact materials which contain chlorine, as compared to otherwise similar materials which contain some other halogen, is that chlorine, because of its anionic size, better fits into the active sites of the metal oxide matrix than do such other halogens. Also, the anionic charge density of chlorine, as opposed to that of other halogen family members, could be preferred for such contact materials used in the conversion of lower alkanes to higher molecular weight hydrocarbons.

Such compositions will preferably contain about 5 to 20 weight percent of the halogen, on an elemental basis. More preferably, the composition will contain about 8 to 17 weight percent of the halogen. For example, compositions of the invention which contain the halogen chlorine will typically contain chlorine in an amount between about 5 to 20 and, preferably, between about 8 to 17.

In the compositions of the invention, the cationic species of the Group IIIB element, the Group IIA metal and the germanium or gallium will generally be present in an approximate molar or atomic ratio of about 1 mole or atom of the Group IIIB element, to no more than about 3 moles or atoms of the Group IIA metal, to no more than about 4 moles or atoms of germanium or gallium. Preferably, these cationic species will be present in a ratio of about 1 mole of the Group IIIB element to about 0.5-3 moles of the Group IIA element to about 0.5-4 moles of germanium and gallium and, more preferably, these cationic species will be present in a ratio of about 1 mole of the Group IIIB element to about 0.5-3 moles of the Group IIA element to about 0.5-3 moles of germanium and gallium. In one preferred embodiment, these cationic species will be present in an approximate molar or atomic ratio of 1 (Group IIIB): 1.5-2.5 (Group IIA): 0.5-1.5 (germanium or gallium).

In one preferred embodiment of the invention, the Group IIIB element is selected from the group consisting of yttrium, lanthanum, neodymium, samarium and ytterbium, as Group IIIB elements which form oxides that do not have a +4 oxidation state. Our experience has been that accessibility to a higher oxidation state, e.g., an oxidation state of +4, leads to contact materials which have poorer selectivity and are more susceptible to reduction, e.g., are susceptible to reduction to a +3 oxidation state, i.e., are reducible metal oxides, and can thus lead to loss of physical strength or lead to increased carbon oxide formation.

In a particularly preferred embodiment the Group IIIB element cationic species is yttrium, at least in part because of the comparative general availability of yttrium.

In one preferred embodiment of the invention the Group IIA metal cationic species will be either strontium or barium, as contact materials containing strontium or barium, as opposed to similar compositions which instead contain other Group II metals or no Group IIA metals at all, generally exhibit a greater selectivity to higher hydrocarbons when the materials are used in oxidative coupling of lower alkanes. The greater selectivity of the subject compositions containing strontium or barium is believed, at least in part, to result from strontium and barium having a preferred ionic size and basicity, as compared to the other Group IIA metals. It is believed that the ionic size of strontium and barium, as being generally more similar to Group IIIB metals, facilitates their incorporation into the material. In addition, basicity is believed to contribute to the ability of the resulting contact material to perform such as in the ability of the contact material to abstract hydrogen from the methane molecule in the oxidative coupling of methane, for example.

One preferred composition of the invention comprises a mixed oxide of cationic species of a) yttrium, b) strontium or barium, and c) zirconium or hafnium, with such compositions containing zirconium and/or hafnium in an amount of no more than about 80 weight percent and, more preferably about 10 to 55 weight percent of the contact material composition and with the approximate molar or atomic ratios of strontium and/or barium to yttrium being in the range of about 0.001 to about 100 (that is about 0.001 to about 100 moles/atoms of strontium and/or barium per mole/atom of yttrium) and, more preferably, in a ratio in the range of about 0.5 to about 3 and, even more preferred, in the range of about 1.5 to about 2.5.

One compositional embodiment of the invention can be represented by the chemical formula:

YₓBa₍₁₋ₓ₎ZrO_{(3+x/2)}

where 0<x<1. It has been found that as the value of x approaches 1, the material is not very active for the oxidative conversion of methane to C₂₊ hydrocarbons. As the value of x approaches 1, a yttrium zirconium oxide is formed. In addition, as the value of x approaches 0, the predominant phase is believed to be barium zirconate perovskite. When the value of x is some intermediate between 0 and 1, preferably when the value of x is in the range of about 0.3 to about 0.7 and, more preferably, when the value of x is about 0.5, the material comprises a mix of three phases, e.g., barium carbonate, barium zirconate and yttrium zirconate, and generally exhibits better rates of oxygen conversion and better selectivity to C₂₊ products in the oxidative conversion of methane to higher hydrocarbons, as compared materials represented by such a chemical formula but having differing values of x.

A composition of the invention comprises a mixed oxide of cationic species of a) yttrium, b) strontium or barium, and c) aluminum, with such compositions containing aluminum in an amount of about 2 to 20 weight percent and, more preferably, about 5 to 10 weight percent of the contact material composition and with yttrium and the strontium and/or barium being present in an approximate molar or atomic ratio of about 1 to about 0.5-3 and, more preferably, in a ratio of about 1 yttrium to about 2 of the Group IIA metal.

Typically, the contact material compositions of the invention will have surface areas generally in the range of about 0.1 m²/gram to about 100 m²/gram and preferably have surface areas in the range of about 1 m²/gram to about 10 m²/gram as such compositions having surface areas in this range generally result in better performance in terms of selectivity and activity, e.g., better C₂₊ selectivity and methane conversion in the oxidative conversion of methane to a higher molecular weight hydrocarbon, as compared to similar compositions with a surface area outside such ranges.

The subject compositions by being substantially free of catalytically effective reducible metals are not susceptible to over-reduction or over-oxidation and the difficulties associated with such changes, as are those compositions containing reducible metal oxides. In addition, the subject contact material compositions are sufficiently hard so that they can be used to form a material that can be fluidized without large losses of material in the form of fines.

The contact material compositions of the invention may be prepared by any suitable method associated with suitable compositions known in the art. Thus, sol-gel preparation techniques, as well as physical mixing techniques, for example, can be used to produce the composition.

The precursor materials resulting from these preparation techniques will typically be calcined at a temperature and for a duration sufficient to lead to a stabilizing interaction among the principal metal cations of the material, whereby solid state transformations typically occur and the material becomes more homogeneous, e.g., "intimately mixed." For example, the precursors can be calcined at 800°C for 8 to 12 hours. In such preparations, the components can be characterized as being intermixed on a microscopic scale (e.g., about 100 micron particle size) and with the components interacting to stabilize and form compound(s) containing more than one of the cationic species. For example, a sol-gel preparation method using barium nitrate, yttria sol and zirconia sol when calcined to 800°C, produced a very homogeneous sample. It is to be understood that such calcination generally results in a stabilizing interaction among the constituents of the material.

Generally, it is preferred to prepare the subject compositions using peroxide, oxide, carbonate and/or hydroxide precursors containing the selected cationic species, which precursors do not contain significant relative amounts of volatile organic species which upon decomposition can act to undesirably increase the surface area of the composition.

It is to be understood that exposure to high temperatures (e.g., about 700°C to about 1,000°C and such as occurs during calcination or, less preferably, in the process use of the material, such as in the oxidative conversion of lower molecular weight alkanes to higher molecular weight hydrocarbons) can and generally will result in desired stabilizing interaction among the constituents of the material. Further, an increase in the homogeneity of the material is observed with the use of the material under oxidative coupling reaction conditions.

Alternatively, such compositions can be prepared via physical mixtures of appropriate metal oxides and/or salts. As will be shown in the examples below, appropriate metal oxides/salts such as yttrium carbonate, barium hydroxide and zirconium oxide can be used. Other appropriate metal oxides/salts include nitrates such as barium nitrate, yttrium nitrate and zirconium dinitrate oxide, carbonates such as barium carbonate, acetates such as barium acetate, as well as materials such as yttrium oxide, for example.

The contact materials of the invention can also be used in oxidative conversion processes to produce dehydrogenated and/or lighter products.

One such application for the compositions of the invention is the oxidative dehydrogenation of dehydrogenatable hydrocarbons and compounds, specifically processing in which the carbon framework of the hydrocarbon feedstock is substantially retained but with the removal of hydrogen therefrom. The process comprises contacting a gas or liquid comprising a dehydrogenatable hydrocarbon or compound with oxygen in the presence of the subject contact material to produce dehydrogenated hydrocarbons and coproduct water. Carbon oxides, i.e., CO and CO₂, and hydrogen can also be formed as by-products of the overall reaction, it being understood that hydrogen by-product can also be formed as a result of water-gas shift reaction.

Dehydrogenatable hydrocarbons include a wide variety of hydrocarbons including C₂₊ alkanes, cycloalkanes, olefins, alkylaromatics, etc., for example. As used herein, dehydrogenatable hydrocarbons are understood to also include various forms of oxygen-containing hydrocarbons such as alcohols (e.g., methanol, ethanol, propanol and butanols) and aldehydes (e.g., ethanol, propanol and butanol) and mixtures thereof with and without other dehydrogenatable hydrocarbons.

The dehydrogenated product depends in part on the feedstock selected. For example, alkanes can be dehydrogenated to form olefins, diolefins, alkynes, etc., olefins can be dehydrogenated to form diolefins, alkynes, etc., aldehydes can be dehydrogenated to form unsaturated aldehydes and alcohols can be dehydrogenated to form aldehydes, for example. One preferred class of feedstock comprises C₂-C₆ alkanes. One preferred process embodiment comprises oxidative dehydrogenation of C₂-C₆ alxanes to form corresponding mono-olefins.

Conditions for oxidative dehydrogenation processing include an operational temperature generally lower than the temperatures preferred for oxidative coupling processing. For example, the temperature for oxidative dehydrogenation processing is generally preferably in the range of about 300°C to about 650°C whereas temperatures for the oxidative coupling of methane are typically preferably greater than 700°C. Operating pressures are not narrowly critical and can range from subatmospheric to pressurized operation. In addition, and at least in part, as a result of operation at lower operating temperatures, operating pressures for oxidative dehydrogenation can be greater than the pressures preferred for the oxidative coupling of lower alkanes such as methane, for example. Lower operating temperatures typically can result in decreased gas phase reaction contributions. Gas phase reactions often are free radical in nature. The collision of radical species with oxygen in the gas phase, however, often leads to the undesirable formation of carbon oxides. While increasing the pressure increases the frequency of gas phase collisions, to the extent that gas phase reaction contributions are decreased as a result of lower operating temperatures usable in oxidative dehydrogenation, the pressure can be increased without a deleterious increase in carbon oxides formation. Generally, for economic reasons, operational pressures of a few hundred pounds per square inch will be preferred for oxidative dehydrogenation processing, as pressures in this range are generally more conducive to economical separation processing and for the optimization of compressor operation and cost.

In oxidative dehydrogenation processing, the ratio of the partial pressure of the dehydrogenatable hydrocarbon feedstock to the partial pressure of the feed oxygen is preferably in the range of from about 0.5:1 to about 40:1 and, more preferably, in the range of from about 1:1 to about 20:1.

The lower limit of the ratio of hydrocarbon feedstock to feed oxygen is generally limited by the flammability range for the feed blend. While some fluid bed reactors can operate with flammable feed blends, such operation is generally undesirable due to safety concerns. In turn, as low conversions per pass typically results in either the unproductive use of feed or to the typically costly recycle of unreacted feed, the upper limit of hydrocarbon to oxygen ratio is generally limited by the need to attain acceptable per pass conversion rates of feed.

As compared to conventional dehydrogenation processing, oxidative dehydrogenation processing advantageously can result in higher conversions, operate at higher pressures without thermodynamic considerations limiting the conversion per pass, generally proceeds through exothermic reactions and is not limited by coke formation as is typically found in conventional dehydrogenation processing. In addition, the exothermic nature of the reactions involved in oxidative dehydrogenation processing can provide at least a significant portion of the heat necessary to bring the reactants up to the desired operating temperature and keep the reactants at the operating temperature during reaction, thus simplifying the heat transfer equipment needed in the operation of the process.

Conventional dehydrogenation is limited by thermodynamic considerations and typically results in the production of hydrogen and a dehydrogenated product. The amount of hydrogen present limits the equilibrium conversion to dehydrogenated product since hydrogen and the dehydrogenated product can react, e.g., rehydrogenate the dehydrogenated product. As conventional dehydrogenation reactions are generally endothermic in nature, increasing the system operating temperature can serve to reduce the effect of such an equilibrium limitation. As the endothermic reactions occur, the temperature in the system decreases until an equilibrium limitation is reached. Significant process system complications result when the operating system is required to provide the amounts of heat required to counter equilibrium limitations and the endothermic nature of the conventional dehydrogenation reaction. In addition, as the system operating temperature is increased, the amount or extent of carbon or coke formation in such processing can become more significant. Consequently, most commercial conventional dehydrogenation processes involve coke removal steps which are typically relatively costly.

While the oxidative dehydrogenation processing of the invention does not result in significant coke formation, the processing does produce at least some carbon oxides. Generally, conventional dehydrogenation processing does not produce such carbon oxides. In the manufacture and production of transportation fuels or fuel additives, the carbon oxides and hydrogen coproduced with the dehydrogenated products can be used or further processed, such as through Fischer-Tropsch processing, such as is applicable to the processing of synthesis gas (also commonly referred to as "syngas"), e.g., a mixture of H₂ and CO, with or without the presence of CO₂. Furthermore, for continuous operation of non-chemical grade olefin production, such as in the manufacture of fuel components, carbon oxide formation is much preferred over coke formation and the problems attendant coke removal processing.

Another specific application for the compositions of this invention is the oxidative cracking of crackable hydrocarbons and compounds. The process comprises contacting a gas or liquid comprising a crackable hydrocarbon or compound with oxygen in the presence of the subject contact material to produce cracked hydrocarbons/compounds. In addition, water and carbon oxides (i.e., carbon monoxide and/or carbon dioxide) as well as some hydrogen, will also typically be produced.

Crackable hydrocarbons include a wide variety of hydrocarbons including C₃+ alkanes, cycloalkanes, alkyl aromatics, etc., for example.

The cracked product depends, in part, on the feedstock selected. Usually, the cracked product will contain olefinic hydrocarbons, e.g., molecules having carbon-carbon double bonds. One preferred class of feedstock comprises C₃-C₆ alkanes. One preferred process embodiment comprises oxidative cracking of C₃-C₆ alkanes to form a cracked product including ethane, ethene, propane, propene, butane and butenes, for example.

Oxidative cracking processing differs significantly from conventional steam cracking processing. Primary areas of difference include heat balances, the formation of carbon oxides and desired unit operation conditions. For example, oxidative cracking processing is an exothermic process which can provide heat needed to raise the temperature of the reactants to the desired cracking operating temperature and to keep the reactants at a desired temperature range during processing, thus simplifying the heat transfer equipment needed for such processing. Generally, oxidative cracking processing has an operational temperature towards the higher end of the operational temperature range for oxidative dehydrogenation processing but less than that of oxidative coupling processing. For example, the operational temperature for oxidative cracking processing is generally preferably in the range of about 450°C to about 650°C with an operational temperature in the range of about 500°C to about 550°C being generally more preferred.

In addition, oxidative cracking is an exothermic process. The heat so generated can be used to heat the reactants to the desired operating temperature and keep the reactants at the operating temperature during reaction, thus simplifying the heat transfer equipment needed in the operation of the process. Generally, the hydrocarbon to oxygen ratios for use in oxidative cracking processing are in the same general range as those previously identified for use in oxidative dehydrogenation processing and are typically limited by the same factors of flammability limits and the economic need for an adequate per pass conversion rate.

In addition, other forms or modes of hydrocarbon cracking typically result in the formation of coke. Generally, such coke can act to foul heat exchange surfaces, e.g., in conventional tubular cracking reactors, coke fouling the heat exchange surfaces generally will serve to decrease the rate of heat transfer. Also, as detailed above, carbon/coke formation and the removal of such carbon/coke typically results in a loss in process productivity. In contrast, in oxidative cracking processing, carbon oxides are formed as opposed to carbon/coke formation. The carbon oxides so formed can be processed as addressed above in the description of oxidative dehydrogenation.

Since the oxidatively driven reactions are not as limited by equilibrium considerations, oxidative cracking processing can be operated at higher pressures than conventional cracking reactions. Higher pressures, such as operating pressures of a few hundred pounds per square inch, are desirable as such high pressures can result in increased efficiency in operating downstream separation processes and in matching desirable operating pressures for downstream conversion processes. Preferred operating pressure ranges generally range from about 0.5 atmospheres absolute to about 100 atmospheres absolute, with operating pressures in the range of about 2 to about 50 atmospheres absolute generally being more preferred. It is understood that the operation of gas reaction processes at elevated pressures results in reducing the size of processing vessels required and thus generally reduces the cost of the vessels and of the associated equipment for compression of products. In cracking processing, the volumes of cracked products as gases are greater than the volumes of the reactants as gases. In such processing, it is generally less expensive to compress the reactants and operate the reactor under pressure rather than compress the gases from a lower pressure reactor.

As will be appreciated from the above discussion of dehydrogenated products resulting from the oxidative dehydrogenation of a dehydrogenatable hydrocarbon and cracked products resulting from the oxidative cracking of a crackable hydrocarbon, such dehydrogenated products can and frequently do contain at least some cracked hydrocarbons and such cracked products can and frequently do contain at least some dehydrogenated hydrocarbons.

It is also to be understood that the contact material of the invention can also have applicability in non-oxidative dehydrogenation and non-oxidative cracking, that is in dehydrogenation and cracking in the substantial absence of oxygen. Such non-oxidative processing is generally under similar reaction conditions as those generally stated above for corresponding oxidative conversion processing.

The contact materials and the processes utilizing the subject contact materials illustratively disclosed herein can suitably be practiced in the absence of any component or ingredient or process step, respectively, which is not specifically disclosed herein.

The present invention is described in further detail in connection with the following examples which illustrate various aspects involved in the practice of the invention. It is to be understood that all changes that come within the spirit of the invention are desired to be protected and thus the invention is not to be construed as limited by these examples.

### EXAMPLES

### Example 1

Preparation by physical mixture of a contact material having the nominal composition YBa₂Zr₃Oₓ, where x = a molar amount necessary for the contact material composition to be at stoichiometric balance, for the nominal composition, x = 9.5.

Yttrium carbonate, Y₂(CO₃)₃·3H₂O (6.18 gm, 0.015 mole), was physically mixed using a mortar and pestle with barium hydroxide, Ba(OH)₂·8H₂O (19.12 gm, 0.0606 moles), and zirconium oxide, ZrO₂ (11.10 gm, .0909 mole). The waters of hydration were enough to make the precursors turn into a sticky paste when ground together. This material was placed in MgO clay crucibles and calcined in air as follows: The sample was placed in a programmable electric furnace and heated to 400°C by increasing the temperature at the rate of 2°C/min, then maintained at 400°C for 1 hour, followed by heating to 800°C by increasing the temperature at the rate of 2°C/min, then maintained at 800°C for 5 hours, and then finally cooled. A tan colored powder was recovered that was determined by XRD to have the following crystalline phases: BaCO₃ 5-378, and ZrO₂ 36-420, 24-1164 as well as poorly crystalline barium zirconate. (The numbers refer to the X-ray file numbers given to known materials cataloged in the Powder Diffraction File). The yttrium phases remained amorphous. Surface area was determined to be 2.6 m²/gm by Kr digisorb analysis. The average pore radius was 4,038A with a bulk density of 1.01 gm/ml as determined by Hg porosimetry.

The contact material was sieved to 80/100 mesh and diluted 10:1 with 30/50 alpha alumina.

### Example 2

Preparation of a sol-gel mixture, contact material having the nominal composition YBa₂Zr₃Oₓ, where x = a molar amount necessary for the contact material composition to be at stoichiometric balance, for the nominal composition, x = 9.5.

Barium carbonate, (31.638 gm, 0.1603 mole) was weighed into a 1 liter beaker along with 200 ml of distilled water. This slurry was heated to 75°C and concentrated nitric acid was added dropwise until no more CO₂ was given off and all the solid BaCO₃ dissolved into solution as Ba(NO₃)₂. The resulting pH was near 1 at 50°C. To this solution was added 64.65 gm of cloudy yttria sol (Nyacol, 14% solids) which was added dropwise. This caused the solution to become more cloudy and viscous and brought the pH up to 6-7. Finally a clear ZrO₂ sol (148.16 gm, Nyacol, 20% solids) was added causing the solution to become more clear yet more viscous. The pH remained at 7 at 50°C and after a minute the sol turned into a thick clear gel while cooling to room temperature. After placing the gel in a hood to dry overnight, it was further dried in a vacuum oven with air purge for 2 days at 85-100°C and at 25 inches of water pressure. A homogeneous crystalline white powder was formed which was placed in a quartz crucible and calcined in air as follows: The sample was placed in a programmable electric furnace and heated at 5°C/min to 400°C, held at that temperature for 1 hour, heated at 2° C/min to 800°C, held at that temperature for 5 hours, then cooled. By XRD the material was highly crystalline and predominantly a new Ba(Y)ZrO₃ perovskite where Y₂O₃ is in solid solution with BaZrO₃. The cubic unit cell has a dimension of 4.21801Å, 36 standard deviation larger than the known BaZrO₃ whose cell dimension is 4.18150A. This phase remains after the catalyst has been used. A minor phase of BaCO₃ was also present. Again no crystalline Y₂O₃ was observed. The bulk density was 1.8 gm/ml and the surface area was only 0.27 m²/gm.

### Example 3

Quantification of the amount of yttria incorporated into the Ba(Y)ZrO₃ perovskite of the contact material of Example 2.

The contact material of Example 2 was analyzed by scanning transmission electron microscopy-energy dispersive X-ray (STEM-EDX). The sample was first ground to a fine powder. The powder material was mixed with an epoxy resin embedding material and then allowed to harden. After hardening, the material was placed in a microtome wherein a diamond knife was used to slice very thin sections from the embedded material. Two phases (phases "B" and "C") of Ba(Y)ZrO₃ were observed. Phase "B" comprised polygonal crystallite clusters and contained approximately 2.4 atomic % Y in BaZrO₃ Phase "C" comprised fine particle clusters and contained 6 to 10 atomic % Y in BaZrO₃. The only other phase detected by STEM-EDX, (phase "A"), comprised dense solid particles, did not contain Ba and had a composition of 6.5 atomic % Y and 27.4 atomic % Zr with the balance being oxygen.

See Table 1 for the STEM-EDX results.

**TABLE 1**

| STEM-EDX (atom %) | | | | | |
|---|---|---|---|---|---|
| | Sample area | Y (as Y₂O₃) | Ba (as BaO) | Zr (as ZrO₂) | O(by diff) |
| Phase "A" | 1 | 6.5 | 1.3 | 27.1 | 65.2 |
| | 2 | 6.6 | 0.4 | 27.6 | 65.4 |
| | | | | | |
| Phase "B" | 3 | 2.4 | 18.3 | 19.1 | 60.2 |
| | 4 | 2.5 | 18.0 | 19.2 | 60.2 |
| | 5 | 2.2 | 18.0 | 19.4 | 60.3 |
| | 6 | 2.4 | 18.4 | 19.1 | 60.1 |
| | 7 | 4.0 | 16.6 | 18.9 | 60.5 |
| | 8 | 2.4 | 18.2 | 19.2 | 60.2 |
| | 9 | 2.4 | 17.9 | 19.4 | 60.3 |
| | | | | | |
| Phase "C" | 10 | 10.2 | 15.4 | 14.6 | 59.8 |
| | 11 | 5.9 | 20.4 | 14.8 | 58.9 |
| | | | | | |

| BaZrO₃ | | | | | |
|---|---|---|---|---|---|
| reference sample | 12 | 0.1 | 18.8 | 20.7 | 60.4 |
| | 13 | 0.5 | 18.4 | 20.7 | 60.5 |
| | 14 | 0.2 | 18.8 | 20.6 | 60.4 |
| | 15 | 0.0 | 18.5 | 21.0 | 60.5 |
| | 16 | 0.0 | 18.5 | 20.4 | 60.2 |
| | 17 | 0.0 | 14.1 | 23.9 | 62.0 |
| | 18 | 0.0 | 19.3 | 20.4 | 60.2 |
| | | | | | |
| stoichiometric values | | | 20.0 | 20.0 | 60.0 |

### Example 5

The contact material of Example 1 was tested for oxidative conversion of methane to higher hydrocarbons.

The contact material was sieved to 80-100 mesh, diluted 10:1 with inert 30-50 mesh alpha alumina diluent and loaded in a plug flow reactor containing a three zone electric tube furnace that heats a 14 mm I.D., 16 mm O.D. quartz tube with a 4 mm O.D. thermowell. Feedstock gas blends were 47% CH₄, 4.7% O₂ with the balance being N₂. Nitrogen was used as an internal standard for the gas analysis which was done with an on-line gas chromatograph. Mass balances of 100 +/- 1% were obtained. Relative feed rates of 72,000 standard cc per hour per cc contact material were used unless otherwise stated.

See Table 2 for reaction conditions and product selectivities.

**TABLE 2**

| REACTION CONDITIONS | | | |
|---|---|---|---|
| Temperature °C (Avg.) | 697.0 | 748.0 | 801.0 |
| SV (1/hr) | 72375 | 72375 | 72375 |
| CH₄/O₂ mole ratio | 10.491 | 10.491 | 10.491 |
| O₂/CH₄ mole ratio | .095 | .095 | .095 |
| O₂ conv., mole % | 28.49 | 51.82 | 85.68 |
| CH₄ conv., mole % (1) | 3.51 | 7.98 | 14.44 |
| CH₄ conv., mole % (2) | 3.72 | 8.29 | 14.44 |
| Res. time (sec) | .005 | .005 | .005 |

| SELECTIVITIES, mole % | | | |
|---|---|---|---|
| CO | 19.67 | 9.50 | 5.03 |
| CO₂ | 27.42 | 20.05 | 16.27 |
| C₂H₄ | 5.88 | 16.33 | 30.19 |
| C₂H₆ | 45.68 | 50.32 | 42.97 |
| C₂H₂ | .00 | .00 | .11 |
| C₃ 's | 1.35 | 3.80 | 5.16 |
| C₄'S | - | - | 0.26 |
| | | | |
| Sel. to C₂₊ | 52.91 | 70.45 | 78.70 |
| | | | |
| C₂H₄/C₂H₆ | .13 | .32 | .70 |
| H₂/CO | 2.66 | 3.17 | 4.21 |
| CO/CO₂ | .72 | .47 | .31 |

| | | | |
|---|---|---|---|
| (1) CH₄ conversion calculated from CH₄ in minus CH₄ out. | | | |
| (2) CH₄ conversion calculated from carbon in products. | | | |

### Example 6

The contact material of Example 2 was sieved to 80-100 mesh, diluted with inert 30-50 mesh alpha alumina diluent in a ratio of 20:1, rather than at a 10:1 ratio as used in Example 5, because of the higher density of the material of Example 2 and tested in the same manner as described in Example 5.

See Table 3 for reaction conditions and product selectivities.

**TABLE 3**

| REACTION CONDITIONS | | | | | |
|---|---|---|---|---|---|
| Temperature °C (Avg.) | 701.0 | 754.0 | 794.0 | 836.0 | 862.0 |
| SV (1/hr) | 73491 | 73491 | 73491 | 73491 | 73491 |
| CH₄/O₂ mole ratio | 11.124 | 11.124 | 11.124 | 11.124 | 11.124 |
| O₂/CH₄ mole ratio | .090 | .090 | .090 | .090 | .090 |
| O₂ conv., mole % | 21.35 | 41.00 | 69.21 | 92.63 | 98.13 |
| CH₄ conv., mole % (1) | 2.93 | 6.57 | 11.39 | 14.58 | 15.15 |
| CH₄ conv., mole % (2) | 2.73 | 6.52 | 11.18 | 14.70 | 15.27 |
| Res. time (sec) | .005 | .005 | .005 | .005 | .005 |

| SELECTIVITIES, mole % | | | | | |
|---|---|---|---|---|---|
| CO | 23.77 | 12.57 | 8.67 | 7.10 | 5.38 |
| CO₂ | 22.49 | 17.05 | 15.81 | 16.13 | 17.31 |
| C₂H₄ | 5.94 | 17.38 | 30.51 | 39.10 | 42.97 |
| C₂H₆ | 46.55 | 49.63 | 40.87 | 31.99 | 28.44 |
| C₂H₂ | - | - | 0.14 | 0.34 | 0.64 |
| C₃'s | 1.24 | 3.36 | 4.00 | 4.75 | 4.37 |
| C₄'s | - | - | - | 0.59 | 0.87 |
| | | | | | |
| Sel. to C₂₊ | 53.73 | 70.37 | 75.51 | 76.77 | 77.31 |
| | | | | | |
| C₂H₄/C₂H₆ | .13 | .35 | .75 | 1.22 | 1.51 |
| H₂/CO | .00 | .00 | .00 | .00 | .00 |
| CO/CO₂ | 1.06 | .74 | .55 | .44 | .31 |

| | | | | | |
|---|---|---|---|---|---|
| (1) CH₄ conversion calculated from CH₄ in minus CH₄ out. | | | | | |
| (2) CH₄ conversion calculated from carbon in products. | | | | | |

### Results:

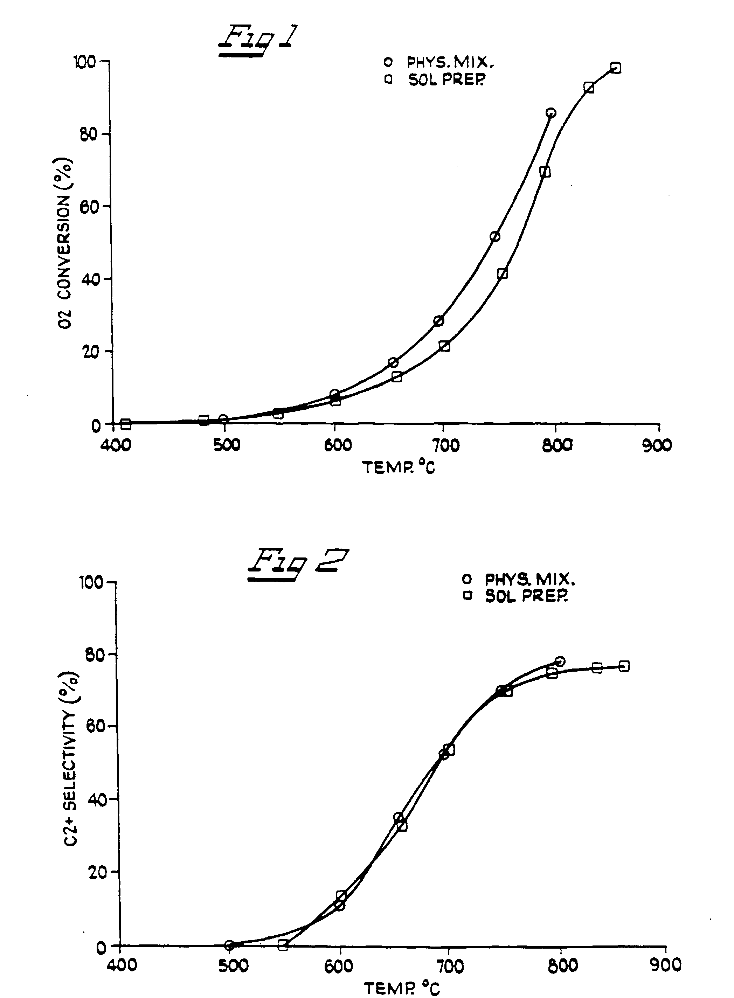

Figure 1 shows the conversion of oxygen varies with respect to reaction temperature and Figure 2 shows the effect on C₂₊ selectivity with varying reaction temperature realized with the use of the contact material of Example 1 (i.e., physical mixture) in Example 5 and with the use of the contact material of Example 2 (i.e., sol preparation) in Example 6.

Selectivities to carbon products and CH₄ conversions were calculated in the following manner:
(a) carbon selectivities were defined as the number of moles of carbon in a desired/specified product component relative to the total number of moles of carbon in all detected products and calculated accordingly.
(b) CH₄ conversion was determined by differences between the amount of CH₄ in the outlet and inlet (molar rates) and also by carbon balance, by the number of moles of product formed (assuming no carbon accumulation).

Deviations of the carbon mass balance from 100% was indicated by a difference in the two measures of CH₄ conversion. Since the reaction is oxygen limited and oxygen is a relatively expensive reactant to produce and process (e.g., separating oxygen from other gaseous species is generally relatively costly), the efficient utilization of oxygen is generally desired. Therefore, oxygen is usually completely consumed under optimal reaction conditions. Oxygen conversion climbed to 86% at 800°C for the physical mixture and 98% at 862°C for the sol-gel mixture. The slight decrease in activity of the sol preparation could be due to the lower surface area and higher bulk density compared to the physically mixed contact material. Selectivities to C₂₊ hydrocarbons are similar for the contact materials of Examples 1 and 2 approaching 79-80% at 800° to 860°C. The only other major products were CO₂ and small amounts of CO.

### Examples 7-12

Activity maintenance, and effect of total oxygen conversion and nitrogen dilution.

### Examples 7 and 8

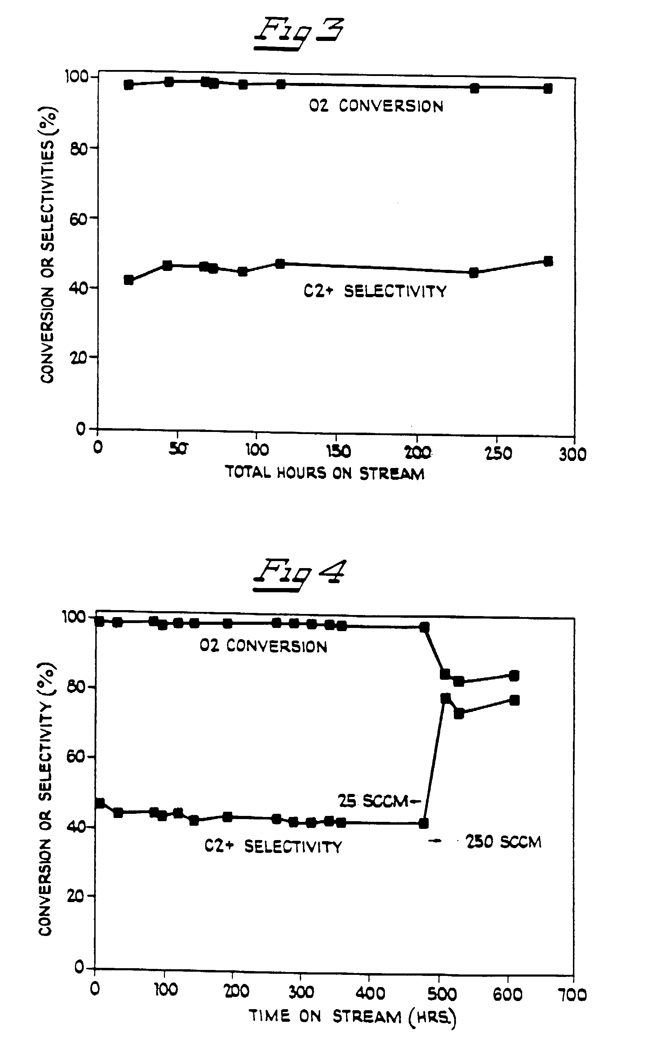

In Examples 7 and 8 the contact materials of Examples 1 and 2 in 0.05 gram samples, respectively, were each tested at 850°C for oxidative conversion of methane to higher hydrocarbons for periods of time on stream of over 250 hours and 450 hours, respectively, at close to total oxygen conversion (e.g., a feed gas flow rate of 25 standard cubic centimeters per minute (sccm)) in order to determine if the contact materials degrade under such conditions. In this testing, a feed gas blend of 40% methane, 4% oxygen, and balance of nitrogen was passed over the contact material at the specified feed rate. Oxygen conversion and C₂₊ selectivities (both in percents) versus total time on stream for the testing of the contact materials of Examples 2 and 1 are shown in Figures 3 and 4, respectively.

As can be seen in Figure 3 and 4, no deactivation was observed for either the contact material of Example 1 or 2 during this time period. This is in contrast to similar contact materials, but which materials contain reducible metals, e.g., tin, which convert into a combustion catalyst under similar conditions.

### Example 9

After a time on stream of over 450 hours, the testing of the contact material of Example 1 for the oxidative conversion of methane to higher hydrocarbons of Example 8, was continued for a period of time of over 100 hours using the same feed gas blend as in Examples 7 and 8 but at a feed rate of 250 sccm.

The results (oxygen conversion and C₂₊ selectivities in percents versus time on stream) are shown in Figure 4.

### Example 10

The contact material of Example 2 was tested in the manner of Examples 7 and 8, but with the specified feed gas blend passed over the contact material at the same 250 sccm feed rate as used in Example 9 with the contact material of Example 1.

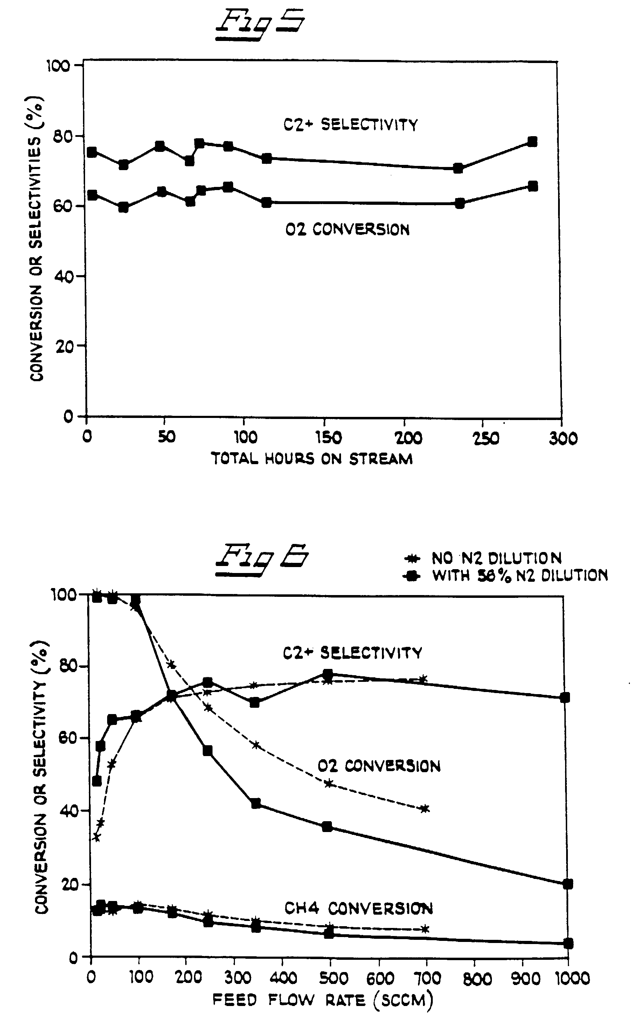

The results (oxygen conversion and C₂₊ selectivities in percents versus time on stream) are shown in Figure 5.

### Additional Discussion of Results of Examples 7-10

Figure 4 shows how an abrupt increase in feed flow rate causes a sharp increase in C₂₊ selectivity and a corresponding decrease in oxygen conversion.

As shown in Figures 4 and 5, increasing the feed flow rate while increasing the C₂₊ selectivity and reducing the oxygen conversion for the contact materials of Examples 1 and 2 did not cause a loss in activity maintenance.

The dependence of C₂₊ selectivity on flow rate at high temperatures is another unique feature of these tested contact materials.

The sol-gel preparation of Example 2 was less active in terms of oxygen conversion per standard weight of catalyst than the physically mixed contact material of Example 1. This may correlate with the higher bulk density of sol-gel preparation and its surface area being an order of magnitude lower than the contact material resulting from the physical mixture process of Example 1.

### Examples 11 and 12

In order to understand how nitrogen dilution affects performance, runs were made in the manner of Examples 7 and 8 but with feed gases with and without nitrogen dilution and at varying feed flow rates. In the case of no nitrogen dilution, the feed gas contained pure methane and oxygen in a 10:1 CH₄:O₂ feed ratio. When nitrogen dilution was used, the feed gas still contained methane and oxygen in a 10:1 CH₄:O₂ ratio with the feed gas containing 40% CH₄, 4% O₂ and 56% N₂.

### Discussion of Results:

Figure 6 shows how the variation of feed flow rate affects various parameters with and without N₂ dilution. Very little change was observed in the C₂₊ selectivity and the CH₄ conversion although oxygen conversion did increase with no N₂ dilution. No problems with coking or loss of temperature control were observed when using pure methane and oxygen in a 10:1 CH₄:O₂ feed ratio. Figure 6 also suggests an optimum flow rate around 200 sccm at 850°C to work at in order to maximize C₂₊ yield.

In order to further test the robustness of the contact material, the contact material was run with pure CH₄ and oxygen for several hours at 850°C after which the oxygen was shut off. Pure CH₄ flowed over the contact material for 5 hours before switching back to the original CH₄:O₂ mixture and the original conversion and selectivity came right back. Before the oxygen was shut off the C₂₊ selectivity was 77%, CH₄ conversion was 8.6% and O₂ conversion was 48%. After the 5 hour treatment with pure CH₄ at 50 and 450 sccm the C₂₊ selectivity was 75%, the CH₄ conversion was 8.9% and the O₂ conversion was 50%. This "shock" of exposure to a reducing atmosphere did not affect the performance of the contact material.

### Example 13

To determine the effect of surface area and pore size distribution, several different preparations were made that resulted in materials having differing surface areas and pore sizes. Table 4 summarizes the different pore sizes and surface areas and the resulting performance for the different preparations in the oxidative conversion of methane to higher hydrocarbons. A feedstock gas blend having a mole ratio of methane to oxygen of 5 to 1 was used. In addition, a relative feed rate of 75 to 300 standard cubic centimeters per minute and a reactor temperature of 825°C-850°C were used.

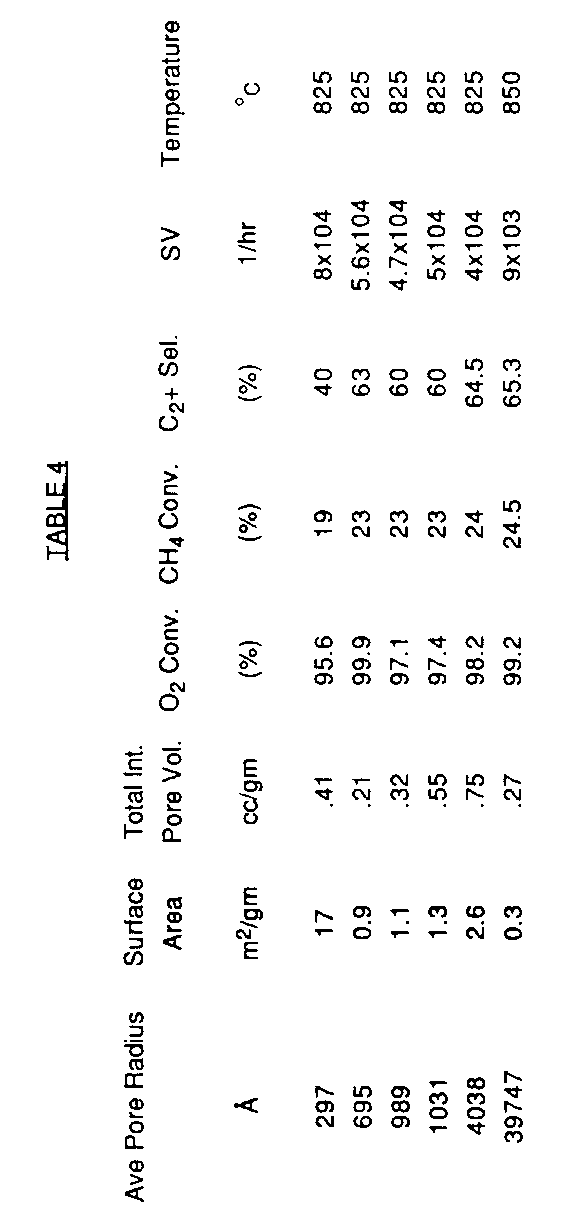

### Descriptions of the different preparations:

### Preparation A - Average Pore Size = 297Å, Surface Area = 17 m²/gm.

Zirconium oxynitrate, ZrO(NO₃)₂·xH₂O (22.3 gm, 7.185 x 10² moles), was added along with yttrium nitrate, Y(NO₃)₃·6H₂O (3.1gm, 8.094 x 10³ moles) and barium nitrate, Ba(NO₃)₂ (4.236gm, 1.621 x 10² moles) to a 500 ml beaker. This mixture of solids was dissolved in distilled water (200 ml) with gentle heating and stirring. An ethylene glycol/citric acid solution was prepared in a separate 250 ml beaker by dissolving 42.1 gm, 0.204 moles of citric acid into 150 ml of ethylene glycol. The citric acid solution was then added slowly to the metal salt solution while stirring. The mixture was then heated to 120°C to drive off NO₂ and complex the metal salt with the citrate anion. After about 16 hours of heating at 120°C, the solution evaporated down to 90 ml and turned darker brown with some white precipitate falling out of solution. The material was vacuum dried for 3 days at 110°C after which it turned to a tan colored solid. This solid was calcined in air using a quartz crucible as follows: The material was heated from 25°C at 5°C/min to 400°C, held at that temperature for 1 hour and then heated at 2°C/min to 800°C. It was held at 800°C for 8 hours before cooling back to 25°C.

### Preparation B - Average Pore Size = 695Å, Surface Area = 0.9 m²/gm.

Zirconium oxide, ZrO₂ powder (26.14 gm, 0.212 moles) was weighed in air into a mortar and pestle along with yttrium carbonate, Y₂(CO₃)·3H₂O (14.43 gm, 0.035 moles) and barium hydroxide, Ba(OH)₂·8H₂O (44.61 gm, 0.1414 moles). Upon grinding the powders together, a sticky paste was formed due to the waters of hydration in several of the precursors. The paste was then placed in a clay crucible and calcined in air as follows: The material was heated from 25°C at 5°C/min to 400°C, held at that temperature for 1 hour and then heated again at 2°C/min to 800°C. The sample was held at 800°C for about 5 hours before cooling to 25°C.

A portion of the calcined material was pelletized by placing 4.6 gm of powder in a 1 1/8 inch die and applying 20,000 psig pressure to it. The pellet was recalcined in air by heating at 3°C/min from 25°C to 800°C and holding at that temperature for 5 hours before cooling to about 25°C.

### Preparation C - Average Pore Size = 989Å, Surface Area = 1.1 m²/gm.

A portion of the calcined material prepared in Preparation B (4.6 gm) was physically mixed with 1.0 gm of Sterotex binder, an organic, animal fat based material. The mixture was pelletized using a 1 1/8 inch die and compressing to 20,000 psig. The pellet was calcined in air by heating at 3°C/min from 25°C to 800°C and holding at that temperature for 5 hours before cooling to about 25°C.

### Preparation D - Average Pore Size = 1031Å, Surface Area = 1.3 m²/gm.

A portion of the calcined material prepared in Preparation B (4.6 gm) was mixed with 2.0 gm of Sterotex powder and pelletized. Pelletizing was done using a 1 1/8 inch die and compressing the powder to 20,000 psig. The pellet was then calcined in air by heating at 3°C/min from 25°C to 800°C and holding at that temperature for 5 hours before cooling to 25°C again.

### Preparation E - Average Pore Size = 4038 Å, Surface Area = 2.6 m²/gm.

A physical mixture was made by grinding together dry powders of zirconia, ZrO₂ (11.2 gm, 0.1815 moles); barium hydroxide, Ba(OH)₂·8H₂O (19.12 gm, 0.06061 moles) and yttrium carbonate, Y₂(CO₃)₃·3H₂O (6.18 gm, 0.015 moles) in a mortar and pestle. The powder turned into a sticky paste upon grinding due to waters of hydration. This paste was transferred to a clay crucible for calcination in air. Calcination was as follows: The sample was heated from 25°C at a rate of 2°C/min to 400°C, held at that temperature for 1 hour and then heated at the rate of 2°C/min to 800°C. It was held at 800°C for 5 hours before cooling to 25°C.

### Preparation F - Average Pore Size = 39747Å, Surface Area = 0.3 m²/gm.

Distilled water (200 ml) was heated to 85°C in a 500 ml beaker. Barium nitrate, Ba(NO₃)₂ (10.000 gm, 0.03824 moles) was dissolved into the water with stirring followed by yttrium nitrate, Y(NO₃)₃·6H₂O (5.26 gm, 0.01914 moles) and zirconium oxynitrate, ZrO(NO₃)₂·xH₂O (16.66 gm, 0.0574 moles). After all three salts were dissolved into the water the pH was about 1. Concentrated NH₄OH (39 ml) was added dropwise to the warm solution causing a white precipitate to form. At a pH of 6 the solution became more viscous. Continued addition of NH₄OH brought the pH up to 10 after which the thickened slurry was evaporated to dryness using a vacuum oven. The dry powder was placed into a yttria stabilized zirconia crucible and calcined in air as follows: The sample was heated from 25°C at 4°C/min to 400°C, held at that temperature for 1 hour and then heated at the rate of 2°C/min to 800°C. It was held at 800°C for 5 hours and then cooled to 25°C. Hard, tan colored particles were collected from the crucible.

### Results:

An increase in activity and a decrease in C₂₊ selectivity was associated with an increase in the surface area of the contact material. The average pore radius steadily decreased as surface area increased. Also, combustion product selectivities (e.g., selectivities for CO and CO₂) increased with the higher surface area materials of the invention.

### Comparative Example 1

### Catalyst of 1% Sr/La₂O₃

A catalyst of 1% Sr/La₂O₃ was prepared by an incipient wetness technique wherein an aqueous strontium nitrate was added dropwise to a dried La₂O₃ powder in the following manner:

An incipient wetness study showed that 0.478 grams of H₂O was needed to impregnate 1.0 gram of La₂O₃ to incipient wetness.

A 2000.0 gram sample of La₂O₃, which had been calcined in flowing air at 500°C for 2 hours, was impregnated to incipient wetness with 956 grams of water containing 48.31 grams of dissolved Sr(NO₃)₂. The resulting material was allowed to sit at room temperature for 2 1/2 hours and then dried overnight at 250°F (121°C).

The dried material was spread thinly and calcined in flowing air at 1112°F (600°C) for 4 hours.

### Comparative Example 2

### Catalyst of 1% Ba/Y₂O₃

A catalyst of 1% Ba/Y₂O₃ was prepared by adding an aqueous solution of barium nitrate dropwise to a Y₂O₃ powder. Y₂O₃, in an amount of 30 grams (0.1329 moles), was weighed into a beaker. BaNO₃, in an amount of 0.585 grams (2.18 x 10 - 3 moles), was weighed into a beaker and dissolved in 20.4 ml of distilled water by gently heating the beaker containing the BaNO₃ and distilled water. This solution was then added dropwise to the beaker containing the Y₂O₃ and physically mixed with the Y₂O₃ by means of a magnetic stir bar to ensure homogeneous wetting of the Y₂O₃. After all of the solution had been added, the resulting slurry was transferred to a yttrium stabilized zirconia crucible and calcined in air as follows: The sample-containing crucible was placed in a programmable electric furnace and heated to 400°C by increasing the temperature at the rate of 4°C/min, then maintained at 400°C for 1 hour, followed by heating to 800°C by increasing the temperature at the rate of 2°C/min, then maintained at 800°C for 8 hours, and then finally cooled to room temperature.

### Comparative Example 3

### Catalyst of 8% Ba/Y₂O₃

A catalyst of 8% Ba/Y₂O₃ was prepared by an incipient wetness technique wherein an aqueous solution of barium nitrate was added dropwise to a dried Y₂O₃ powder. Y₂O₃, in an amount of 30 grams (0.1329 moles), was weighed into a beaker and dried overnight in a vacuum oven at 140°C. BaNO₃, in an amount of 5.721 grams (2.19 x 10⁻² moles), was weighed into a beaker and dissolved in 40 ml of distilled water by gently heating the beaker containing the BaNO₃ and distilled water. The BaNO₃ solution was added dropwise to the Y₂O₃ until the wetness point of the material was reached (i.e., after about 20 ml of the BaNO₃ solution had been added) forming a slurry. The slurry was gently heated to dryness and then the balance of the BaNO₃ solution was added dropwise to the dried material. After all the solution had been added, the resulting slurry was transferred to a yttrium stabilized zirconia crucible and calcined in air as follows: The sample-containing crucible was placed in a programmable electric furnace and heated to 400°C by increasing the temperature at the rate of 4°C/min, then maintained at 400°C for 1 hour, followed by heating to 900°C by increasing the temperature at the rate of 2°C/min, then maintained at 900°C for 8 hours, and then finally cooled to room temperature.

The final material was subjected to XRF analysis and found to contain 8% Ba.

### Example 14 and Comparative Examples 4. 5 and 6

### Lifetime testing

The YBa₂Zr₃Oₓ contact material composition of Example 1 and the catalysts of 1% Sr/La₂O₃ (Comparative Example 1), 1% Ba/Y₂O₃ (Comparative Example 2), and 8% Ba/Y₂O₃ (Comparative Example 3), respectively, were tested in a fully automated plug flow microreactor, which utilized a SETCON program to control all reactor heating cycles, gas flows and valve switching. In addition, all the contact material samples in the reactor were diluted with alpha-alumina to permit better heat control, in the ratio of 7 parts diluent per 1 part of contact material.

A gas feedstock containing 30% CH₄ 6% O₂ with the balance being N₂ was used. Reaction conditions included a reaction temperature of 850°C and, as operation at or near 100% O₂ conversion was desired, the feed rate (325 ml/min) was selected to obtain 100% oxygen conversion at the start of the run. (Note: In view of the high cost of pure O₂, maximizing the utilization of O₂ is generally preferred.)

### Results:

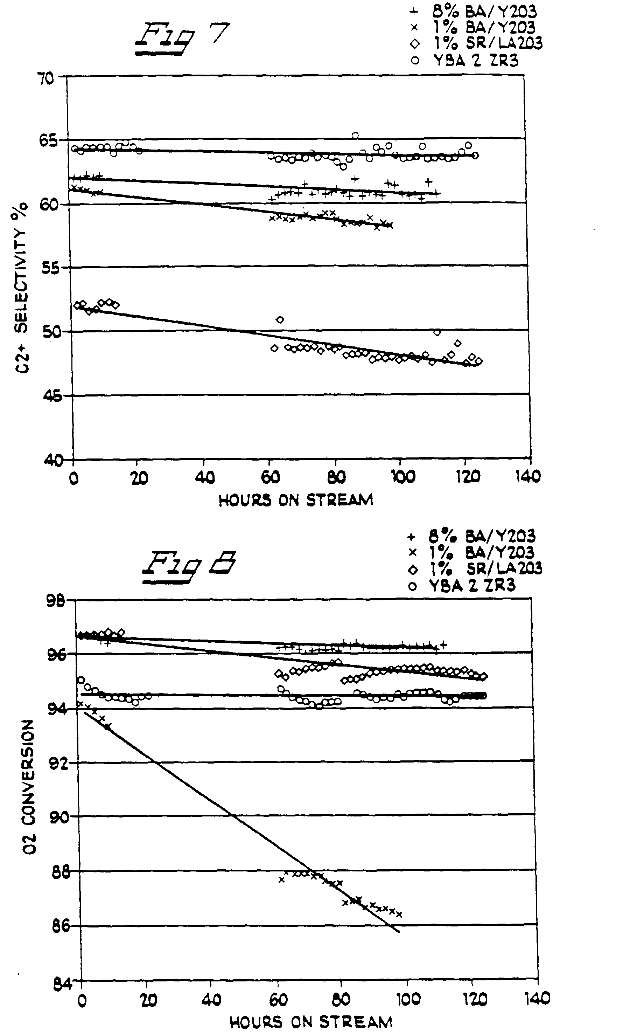

Figures 7 and 8 show the effect on C₂₊ selectivity and oxygen conversion, respectively, with time obtained in Example 14 and Comparative Examples 4, 5 and 6.

For both C₂₊ selectivity and oxygen conversion, the largest declines over time were realized with the 1% Sr/La₂O₃ catalyst of Comparative Example 1. The 1% Ba/Y₂O₃ catalyst of Comparative Example 2 and the 8% Ba/Y₂O₃ catalyst of Comparative Example 3 realized the next greatest deactivation rates. The YBa₂Zr₃Oₓ contact material composition of Example 1 realized the lowest deactivation rate of these tested materials.

### Examples 15 and 16 and

### Comparative Examples 7, 8 and 9 and 10, 11 and 12

### Metal leeching rate testing

The YBa₂Zr₃Oₓ contact material composition of Example 1 and the catalysts of Comparative Examples 1, 2 and 3, respectively, were subjected to bulk metals analysis by X-ray fluorescence in Example 15 and Comparative Examples 7, 8 and 9, respectively.

In addition, after a time on stream of 120 hours, the contact material used in Example 13 and the catalysts used in Comparative Examples 4, 5 and 6, respectively, were also subjected to bulk metals analysis by X-ray fluorescence in Example 16 and Comparative Examples 10, 11 and 12, respectively.

### Results:

Table 5 shows the bulk metal analysis results obtained.

In comparing the used with the fresh materials, 1% Sr/La₂O₃ had lost virtually all of the Sr with 120 hours on stream. 1% Ba/Y₂O₃ had lost over 70% of the Ba with 120 hours on stream. 8% Ba/Y₂O₃ had lost about 12% of the Ba with 120 hours on stream. YBa₂Zr₃Oₓ lost virtually no Ba, Y or Zr with 120 hours on stream under the same reaction conditions.

These examples show that the compositions of the subject invention, even when subjected to the extreme reaction conditions associated with oxidative coupling are not subject to the high rates of volatilization associated with other contact material compositions. This translates into an increased lifetime for the contact material composition, as compared to known oxidative coupling contact materials which undergo substantial metal lose with time on stream.

**TABLE 5**

| Bulk XRF Analysis | | | | |
|---|---|---|---|---|
| | | Sr(Wt.%) | La (Wt.%) | |
| Comp. Ex. 7 | Fresh | 0.98 | 81.5 | |
| Comp. Ex. 10 | Used (120 hr) | 0.005 | 57.8 | |

| | | Ba(Wt.%) | Y (Wt.%) | |
|---|---|---|---|---|
| Comp. Ex. 8 | Fresh | 1.07 | 74.1 | |
| Comp. Ex. 11 | Used (120 hr) | 0.3 | 70.7 | |
| Comp. Ex. 9 | Fresh | 7.5 | 66.8 | |
| Comp. Ex. 12 | Used (120 hr) | 6.7 | 68.9 | |

| | | Ba(Wt.%) | Y (Wt.%) | Zr (Wt.%) |
|---|---|---|---|---|
| Ex. 10 | Fresh | 32.1 | 8.0 | 31.1 |
| Ex. 11 | Used (120 hr) | 32.0 | 8.4 | 40.7 |

### Comparative Example 13

### YBa₂ on alumina support

Twenty grams of a fluidizable alpha alumina (UCI-SHAT-99-16) was calcined for 1 hour at 500°C. Yttrium acetate tetrahydrate, 0.840 grams, was dissolved in water to form a solution. Barium acetate, 0.967 grams, was dissolved in the solution and the resulting solution was diluted to 5.6cc total volume. This solution was added to the dried alumina support under a partial vacuum and then dried at 120°C. The dried material was calcined at 800°C for 4 hours. (Notes: nominally 0.85 wt.% Y, 2.5 wt.% Ba, which corresponds to about 2 Ba to 1 Y on a mole basis)

### Comparative Example 14

### The catalyst of Comparative Example 13 was tested for methane partial oxidation.

A 0.5 gram portion of the catalyst produced in Comparative Example 13 was diluted with 1.00 gram of 30-50 mesh low surface area alpha alumina material and loaded in a quartz reactor, forming a catalyst zone. A feed gas blend of 30% methane, 6% oxygen, and balance of nitrogen was passed over the catalyst at 100 standard cc per minute to give a relative feed rate of 12,000 cc-gas per hour per gram of catalyst. Temperature of the catalyst zone was controlled to 850°C.

### Results:

Initial oxygen conversion of the catalyst was 65% but decreased quickly to about 38%. Selectivity for C₂₊ hydrocarbons at 38% oxygen conversion was about 58% with the hydrocarbons being primarily ethane and ethylene. The balance of the carbon-containing products were carbon oxides.

### Example 17

Preparation of a sol-gel mixture, contact material having the nominal composition YBa₂AlOₓ, where x = a molar amount necessary for the contact material composition to be at stoichiometric balance for the nominal composition, x = 5.

A PHF alumina sol (56.3 grams) containing about 9 weight percent alumina was mixed with 100cc of water. To this mix, 80.7 grams of a yttria colloid (from Hydrocol Corp.) containing about 14 weight percent yttria was added and mixed. During the addition and mixing, no formation of a precipitate was observed.

Barium hydroxide octahydrate, (63.3 grams) was placed in 200cc of water to form a solution and heated to about 40°C to 60°C to aid in the dissolution of the barium hydroxide octahydrate. The barium-containing solution was added to the yttria alumina mix and a thin gel formed. The gel was put in a vacuum oven with 40 inches of vacuum with a small air purge and with the oven set at 120°C for overnight.

The dried material was put in an air-purged muffle furnace at room temperature. The temperature setting on the furnace was set to 800°C and the material was left in the furnace for 3 hours. (Note: The furnace took about 30 minutes to achieve an internal temperature of 800°C)

### Example 18

### Testing of the contact material of Example 17

The contact material of Example 17 was tested in a manner similar to that used in Comparative Example 2 except that a feed rate of 200 standard cc per minute (double the feed rate used in Comparative Example 18) was used and the contact material bed was diluted with 0.5 grams of diluent instead of 1 gram.

### Results:

Oxygen conversion was 100% and methane conversion was 22% to 23%, which is much higher than the methane conversion realized in Comparative Example 14. The selectivity to higher hydrocarbons, e.g., C₂₊ hydrocarbons, was about 61% which is slightly better than that realized in Comparative Example 14. Hydrogen was also produced, but only in quantities sufficient to match that expected from water gas shift reaction. The hydrogen equivalence ratio, i.e., (H₂-CO₂) / (CO+CO₂) was about zero.

In this example, the contact material of the invention wherein alumina is incorporated into the contact material itself (the composition of Example 17) was at least about twice as active as a catalyst prepared by the method of Comparative Example 13 wherein alumina was used as a support.

In addition, hydrogen was produced in sufficient quantities so as to be able to theoretically react with the product carbon dioxide to form carbon monoxide and by-product water with some hydrogen remaining. Such remaining hydrogen can, if desired, be used to convert the carbon monoxide to other useful and desired products, such as through Fischer-Tropsch processing, for example.

A way of showing the amount of hydrogen produced is through an "effective hydrogen to carbon oxides ratio," calculated as (H₂-CO₂)/ (CO+CO₂), on a molar basis. This equation subtracts the amount of CO₂ produced from the amount of hydrogen produced to account for the CO₂ shifting back to CO via the water-gas-shift reaction, i.e., H₂+ CO₂ → CO+H₂O. The amount of hydrogen remaining is then divided by the amount of product CO plus the amount of CO that would result from the shifting back of the produced CO₂.

An effective hydrogen-to-carbon oxides ratio of 2 is near the stoichiometric amount of hydrogen needed to form paraffinic products and water (such as by Fischer Tropsch reactions) or to form methanol. At ratios below 2, carbon will have to be rejected or hydrogen added in order to attain ratios of 2. In practice, gases having low effective hydrogen-to-carbon oxides ratios shift CO to CO₂+H₂ and reject some CO₂. At ratios above 2, additional carbon oxides can be added to the processing stream or the carbon oxides can be added to the processing stream or the carbon oxides can be methanated to methane and water (methanation typically requires a ratio of about 3).

### Example 19

The contact material of Example 17 was tested in a manner similar to that used in Comparative Example 14 except that the methane of oxygen feed gas ratio was increased to 10:1 by using a blend of 40% methane, 4% oxygen, and the balance nitrogen (on a volume basis). Also for this example, the amount of contact material was reduced ten fold so that only 0.05 grams of contact material was used. By so doing, the relative feed ratio was increased ten fold relative to that of Comparative Example 14.

### Results:

With operation at 850°C, oxygen conversion was essentially complete showing that the contact material used in this Example was much more active than the catalyst of Comparative Examples 13 and 14. In Example 19, the selectivity to higher hydrocarbons (C₂₊) was 70% with the balance of carbon being present as carbon oxides. The effective hydrogen to carbon oxide ratio was about 0.4, providing the potential for further conversion of the carbon oxides and hydrogen products.

### Example 20

The contact material of Example 17 was tested as in Example 19 except that the feed rate was doubled. The temperature was also increased to 875°C.

### Results:

Oxygen conversion remained at essentially 100% indicating that the contact material was at least 20 times more active at the same temperature than the catalyst of Comparative Examples 13 and 14. The selectivity to higher hydrocarbons (C₂₊) increased to 75% with the balance of carbon being present as carbon oxides. The effective hydrogen to carbon oxides ratio increased to 0.6. Indicating that potentially more of the carbon oxides produced can be relatively more easily processed to produce more valuable products, as compared to processing resulting in a lower effective hydrogen to carbon oxides ratio.

### Example 21

The contact material of Example 17 was tested in a manner similar to that of Comparative Example 14 except that 0.100 gram of contact material powder was used and the feed gas, in addition the specified percentages of CH₄ and O₂, contained 10 volume % CO₂ with a corresponding reduction in the amount of nitrogen in the feed.

The feed rate was 200 cc per minute. A series of temperatures (800°C, 825°C, 850°C, 875°C and 900°C) were examined. The temperature was maintained at each of the examined levels for a period of 3 hours.

### Results:

At 875°C, oxygen conversion was about 93% showing that the presence of CO₂ in the feed suppressed the activity of the contact material, but the activity of the material was still extremely high even with the relative feed rate of 120,000 cc of feed gas per gram of contact material. Methane conversion was 23% with a selectivity to C₂₊ hydrocarbons of about 60%. Carbon monoxide and a net amount of CO₂ were produced along with hydrogen.

In view of this example, carbon dioxide in the feed can be used to moderate the high activity of this contact material and carbon dioxide does not have to be completely removed from the feed.

At 900°C, complete oxygen conversion was attained with a selectivity to C₂₊ hydrocarbons of 60% and a methane conversion of 23-24% per pass.

### Example 22

### Contact Material Stability

The stability of the contact material of Example 17 under oxidative coupling reaction conditions for methane conversion was examined in the following manner:

A 0.050 gram portion of the contact material of Example 17, in the form of a fine powder of 100-180 mesh, was diluted with an inert diluent solid in a weight ratio of 10:1 and loaded in a quartz reactor, forming a contact material zone. A feed gas blend of 40 volume% methane, 4 volume% oxygen and the balance nitrogen was passed over the contact material-diluent mass at the relative feed rate of 120,000 standard cc-gas per hour per gram of contact material.

A series of operating temperatures (750°C, 775°C, 800°C, 825°C, 850°C and 875°C) for the contact material zone were investigated with the operating temperature held at each of the selected temperatures for about 3 hours followed by a return to room temperature. The highest C₂₊ selectivity was realized at the highest temperature examined, i.e., 875°C.

To examine the stability of the contact material over an extended operating time period, the contact material zone temperature was brought to 875°C and, using a relative feed rate of 240,000 standard cc-gas per hour per gram of contact material, held constant at these operating conditions for 2 days of operation.

### Results:

The stability of the contact material shows that there was no significant change in the performance of the contact material as demonstrated by the maintenance of the C₂₊ selectivity and oxygen and methane conversion levels during the duration of the test period, showing the stable behavior of the contact material. Further, even at the higher feed rate used in this stability testing, the O₂ conversion was essentially 100%, indicating a contact material of very high activity.

### Example 23

### Larger scale sol-gel preparation followed by spray drying

Physical requirements for a fluidizable contact material include appropriate particle size and attrition resistance. Fluidizable contact materials are typically spray dried. A spray dried contact material with a nominal composition of YBa₂AlOₓ, where x = molar amount, necessary for the composition to be at stoichiometric balance was prepared in the following manner:

A yttria sol (51.40 Ibs.) from Nyacol Products Inc. was blended with a PHF alumina sol (40.60 Ibs.) using a homogenizer. A barium hydroxide solution (40.10 Ibs. BaOH/43 l water), heated to 50°C, was added to this mixture while mixing. As a gel thickened, an additional 30 gallons of water were added to the gel mixture to dilute the thickness of the gel. The resulting gel was thoroughly blended with the homogenizer. The material from the homogenizer was spray-dried and collected.

The fines and large particle bulk material were reslurried and spray-dried a second time to increase the total yield of fluidizable contact material. The material was placed directly in an oven at 800°C and calcined in flowing air for 5 hours.

### General Guidelines Re Particle Size:

The average particle size range for the fluidizable material will typically be about 30 to 125 microns, with a median fluidizable particle size of about 72 to 75 microns.

| Typical Size Distribution Particle Size (Microns) | Weight Percent |
|---|---|
| 80+ | 25 |
| 40-80 | 60 |
| 20-40 | 15 |
| 0-20 | ≤1 |

### Example 24

The fluidizable YBa₂AlOₓ contact material of Example 23 was loaded into a fixed bed reactor unit using a gaseous feed stream containing 30 volume% CH₄, 6 volume% O₂, and the balance being N₂ gas and a feed rate of 150,000 cc of feed at ambient conditions per hour per gram of contact material for runs at 750°C and 850°C.

The results are given in Table 6 below.

**TABLE 6**

| | | | |
|---|---|---|---|
| Temperature (°C) | 750 | 800 | 850 |
| Methane Conversion (%) | 18.2 | 21.9 | 21.7 |
| (in-out) | | | |
| Oxygen conversion (%) | 86 | 98 | 99 |
| C₂₊ selectivity (%) | 52.5 | 61.8 | 63.0 |
| CO₂ selectivity (%) | 35 | 32 | 31 |
| CO selectivity (%) | 12 | 6 | 6 |

### Discussion of Results:

The spray dried contact material of Example 23 was highly active as shown by the 99% oxygen conversion realized at 850°C, with a relative feed ratio of 150,000 cc/gm-hr versus the results obtained in Comparative Example 14 in the testing of the material of Comparative Example 13, wherein oxygen conversion was only about 38%.

In addition, selectivity to C₂₊ hydrocarbons for the spray dried contact material of Example 23 was 63%, as compared to the C₂₊ hydrocarbon selectivity of only 58% in Comparative Example 14.

As shown in Example 24, the performance of the spray dried material of Example 23 was comparable (in terms of activity and selectivity) to the sol-gel preparation of Example 17 (tested in Example 18).

### Examples 25 and 26

In these examples, the contact material of Example 17 was used in an oxidative conversion process, using n-butane as a feedstock, to produce dehydrogenated and lighter products.

In both examples, the contact material was tested in a tubular fixed bed quartz reactor with inert alpha alumina packed in the heated pre- and post-contact material zones. The reaction temperature was measured via a quartz thermowell. The feedstock gas was 4 volume% oxygen and 4 volume% n-butane with the balance of the feedstock gas being inert nitrogen. In Example 25, the contact material was tested in a once-through mode of operation, with no recycle.

In Example 26, the contact material was tested with recycle using an external pump between the reactor effluent and inlet streams.

Tables 7 and 8 identify the reaction conditions and conversion and selectivity results, respectively, for Examples 25 and 26.

**TABLE 7**

| REACTION CONDITIONS | | |
|---|---|---|
| | Example 25 | Example 26 |
| Temperature (°C) | 700 | 700 |
| Pressure (atm) | 1 | 1 |
| Feed Rate (sccm) | 100 | 50 |
| Recycle Rate (sccm) | - | 1000 |
| Catalyst Weight (g) | 0.2 | 0.2 |

**TABLE 8**

| CONVERSION AND SELECTIVITY RESULTS | | |
|---|---|---|
| | Example 25 | Example 26 |
| Conversion of n-butane, C% | 66.0 | 77.5 |
| Selectivity, C% | | |
| Carbon Monoxide | 7.5 | 9.9 |
| Carbon Dioxide | 12.1 | 14.0 |
| Methane | 12.2 | 17.7 |
| Ethene | 36.9 | 49.6 |
| Ethane | 1.7 | 2.3 |
| Propene | 23.4 | 6.2 |
| Propane | 0.4 | 0.3 |
| cis-Butene-2 | 0.9 | - |
| trans-Butene-2 | 1.1 | - |
| Butene-1 | 1.9 | ⁻ |
| 1.3-Butadiene | 1.9 | - |
| Selectivity to H₂, mol% | 12.8 | 13.5 |
| Carbon Mass Balance, % | 104.0 | 107.4 |
| (* = mole of hydrogen produced per mole of hydrogen in the converted butane) | | |

### Discussion of Results:

As shown in Table 8, Examples 25 and 26 resulted in significant conversion of n-butane to ethene and propene, which are both high-valued chemical feedstocks. By controlling the mode of operation (e.g., with or without recycle), both n-butane conversion and ethene selectivity can be favorably affected.

### Example 27

### Synthesis of Y(BaCl₂)₂Ge₃O_{y}, where y = a molar amount necessary for the contact material to be at stoichiometric balance, for the nominal composition, y = 7.5.

A physical mixture having the nominal composition Y(BaCl₂)₂Ge₃O_{y} was prepared by physically mixing a yttrium carbonate (Y₂(CO₃)₃·3H₂O, 6.2 gm, 0.015 moles) and barium hydroxide octahydrate (Ba(OH)₂·8H₂O, 19.1 gm, 0.061 moles). Germanium chloride (GeCl₄, 19.3 gm, 0.09 moles) was added dropwise to this physical mixture in air to form a slurry. The resulting slurry was mixed via mortar and pestle resulting in a material having a paste consistency. The paste, which was white in color, was transferred to a crucible and then calcined in air as follows: The sample was heated at the rate of 4°C/min to 800°C and was then maintained at that temperature for 5 hours. X-ray diffraction (XRD) of the calcined material showed several crystalline phases including BaGe₄O₉ and other unidentified phases.

### Example 28

### Synthesis of Y(BaCl₂)₂GeO_{y}, where y = a molar amount necessary for the contact material to be at stoichiometric balance, for the nominal composition, y = 3.5.

A preparation having the nominal composition Y(BaCl₂)₂GeO_{y} was prepared by:

Weighing barium hydroxide octahydrate (Ba(OH)₂·8H₂O, 32.19 gm, 0.102 moles) and yttrium carbonate (Y₂(CO₃)₃·3H₂O, 10.31 gm, 0.025 moles) into a mortar and pestle and grinding them together for several minutes. The mixture was then transferred to a nitrogen filled glove bag where 10.83 gm 0.0505 moles of germanium chloride (GeCl₄) was added dropwise. Mixing these precursors together created a paste that emitted some HCI fumes. The paste was then transferred to a yttria stabilized zirconia crucible for calcination in air. Calcination was as follows: The material was heated at a rate of 4°C/min to 400°C, maintained at that temperature for 60 minutes before heating at a rate of 2°C/min to 800°C where it was maintained for 720 minutes. The sample was then cooled. The major crystalline phase was BaCl₂ and BaCl₂· H₂O, with a previously undisclosed phase of Ba₅Cl₆GeO₄. Minor phases of BaGe₂O₅ and Y (OH)₃ also were present. The unit cell parameters for the Y₂GeO₅ solid solution are a = 10.4329(75) Å, b = 6.7763(26) Å, c = 13.0262 (158) Å. The surface area of the fresh contact material was 2.6 m²/gm.

### Example 29

An alternative method of preparation of the Y(BaCl₂)₂GeO_{y}, where y = 3.5 contact material was used and involved physically mixing in air yttrium carbonate (Y₂(CO₃)₃·3H₂O, 20.61 gm, 0.05 moles) with barium chloride (BaCl₂·H₂O, 48.91 gm, 0.20 moles), and germanium oxide GeO₂, 10.47 gm, 0.10 moles) using a mortar and pestle. After the precursors were ground to a homogeneous white powder the mixture was transferred to a yttria stabilized zirconia crucible for calcination. Calcination was as follows: The sample was heated at a rate of 4°C/min to 400°C, then maintained at that temperature for 60 minutes before increasing the calcination at a rate of 2°C until a temperature of 850°C was attained. The material was maintained at 850°C for 6 hours. The sample was then cooled ballistically as the oven cooled. XRD analysis showed there were seven identifiable phases, BaCl₂·2H₂O, BaGe₄O₉, and Y₂O₃ were all major phases. Intermediate phases were GeO₂, Y₂Ge₂O₇, and BaCl₂. XPS analysis indicates a high binding energy peak for barium at around 783 eV binding energy.

### Example 30

### Preparation of a contact material having the nominal composition of Y(BaCl₂)₂GaO_{y}, where y = a molar amount necessary for the contact material to be at stoichiometric balance, for the nominal composition, y = 3.

Yttrium carbonate (Y₂(CO₃)₃·3H₂O, 8.26 gm, 0.020 moles), barium chloride (BaCl₂·2H₂O, 19.74 gm, 0.08 moles), and gallium nitrate (Ga(NO₃)₃·9H₂O, 10.24 gm, 0.040 moles) were physically mixed in air using a mortar and pestle. After the precursors were ground to a moist paste the mixture was transferred to a yttria stabilized zirconia crucible for calcination. Calcination was as follows: The sample was heated at a rate of 4°C/min to 400°C, then maintained at that temperature for 60 minutes before increasing the temperature at a rate of 2°C/min to 850°C, and then maintained at that temperature for 720 minutes. The sample was then cooled ballistically as the oven cooled.

### Example 31

### Preparation of a contact material having the nominal composition Y(BaBr₂)₂GeO_{y}, where y = a molar amount necessary for the contact material to be at stoichiometric balance, for the nominal composition, y = 3.5.

Yttrium carbonate (Y₂(CO₃)₃·3H₂O, 6.18 gm, 0.015 moles), barium bromide (BaBr₂, 17.83 gm, 0.06 moles), and germanium oxide (GeO₂, 3.14 gm, 0.03 moles) were physically mixed in air using a mortar and pestle. After the precursors were ground to a moist paste the mixture was transferred to a yttria stabilized zirconia crucible for calcination Calcination was as follows: The sample was heated at a rate of 4°C/min to 400°C, then maintained at that temperature for 60 minutes before increasing the temperature at a rate of 2°C/min to 850°C, and then maintained at that temperature for 800 minutes. The sample was then cooled ballistically as the oven cooled.

### Examples 32 - 36

### Catalytic Activity for Methane Oxidative Coupling Examples

The contact materials of Examples 27-31, respectively, were tested for catalytic activity. Feed mixtures were from premixed tanks containing either a feed mixture having a targeted methane to oxygen mole ratio of 2 to 1 and comprising 7% O₂, 14% CH₄ and the balance of N₂, or a feed mixture having a targeted methane to oxygen mole ratio of 5 to 1 and comprising 6% O₂, 30% CH₄ with the balance of nitrogen. The contact materials were crushed and sieved 80/120 (e.g., particles pass through 80 mesh screen but are retained on 120 mesh screen), and mixed with an appropriate amount of alpha alumina diluent that was 30/50 mesh to result in a material just providing about 100% O₂ conversion. Many of these contact materials required no alumina diluent since they were not very active compared to their nonhalide-containing counterparts.

The table below identifies actual methane to oxygen ratio of the feed mixture used in each of these Examples and the ratio of contact material to diluent (CM/D) in those Examples where diluent was used.

| Example | Contact Material of Example | Feed Mixture (actual CH₄/O₂ Mole Ratio) | CM/D |
|---|---|---|---|
| 32 | 1 | 5.236 | 4.42/2.3 |
| 33 | 2 | 5.242 | 2.23/1.2 |
| 34 | 3 | 1.941 | 3.79/2.0 |
| 35 | 4 | 4.828 | NONE |
| 36 | 5 | 4.466 | NONE |

The gas flows were controlled by Brooks mass flow controllers which were controlled by a SETCON program. In fact, the entire reactor system was automated by using SETCON to control all reactor heating cycles, gas flows, gas chromatograph injections, and valve switching. Product analysis was done using an H.P. 5890 gas chromatograph in a multiple column configuration. Multichrome was used for integration of the G.C. peaks and a 1032 database for a conversion, selectivity, and mass balance calculations. The results are given in Tables 9 - 13, respectively, below.

**TABLE 9**

| REACTION CONDITIONS | | | | |
|---|---|---|---|---|
| Hours into run | 9:20 | 10:36 | 11:52 | 13:08 |
| Temp. C (Avg.) | 800.0 | 799.9 | 850.1 | 850.0 |
| SV (1/hr.) | 5350 | 7120 | 5350 | 7120 |
| O₂ conv., mole % | 54.403 | 42.131 | 93.256 | 99.254 |
| CH₄ conv. mole %(1) | 16.35 | 13.66 | 24.04 | 16.64 |
| CH₄ conv. mole %(2) | 15.23 | 12.95 | 22.25 | 15.06 |
| Res. time (sec.) | 0.068 | 0.052 | 0.065 | 0.049 |

| SELECTIVITIES, mole % | | | | |
|---|---|---|---|---|
| CO | 12.63 | 10.97 | 16.29 | 28.82 |
| CO₂ | 9.30 | 8.15 | 13.57 | 54.37 |
| C₂H₄ | 49.47 | 46.57 | 52.34 | 13.29 |
| C₂H₆ | 22.74 | 29.11 | 10.06 | 1.11 |
| C₂H₂ | 0.00 | 0.00 | 1.77 | 1.11 |
| C₃H₈ | 0.51 | 0.69 | 0.00 | 0.00 |
| C₃H₆ | 4.57 | 3.96 | 5.70 | 1.30 |
| C₄'s | 0.78 | 0.55 | 0.25 | 0.00 |
| C₂₊ | 78.07 | 80.89 | 70.13 | 16.81 |
| | | | | |
| C₂H₄/C₂H₆ | 2.17 | 1.60 | 5.20 | 11.99 |
| H₂/CO | 0.00 | 0.00 | 0.00 | 0.00 |
| CO/CO₂ | 1.36 | 1.35 | 1.20 | 0.53 |

| | | | | |
|---|---|---|---|---|
| (1) CH₄ conversion calculated from CH₄ in minus CH₄ out. | | | | |
| (2) CH₄ conversion calculated from carbon in products. | | | | |

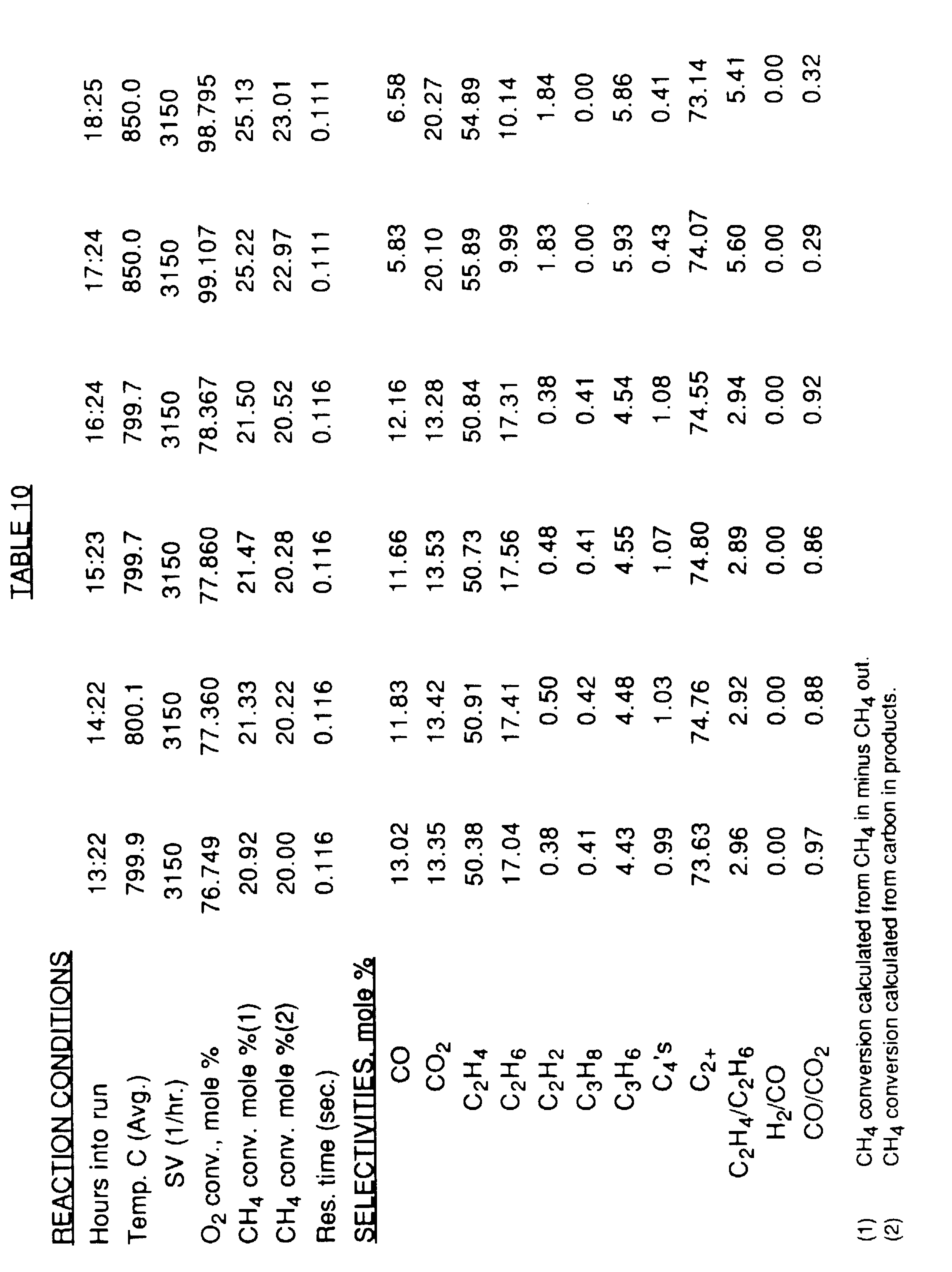

**TABLE 11**

| REACTION CONDITIONS | | | | |
|---|---|---|---|---|
| Hours into run | 11:46 | 13:28 | 15:09 | 16:50 |
| Temp. C (Avg.) | 899.7 | 799.8 | 849.9 | 850.1 |
| Flow Rate (g/hr.) | 149.1 | 149.3 | 149.3 | 99.4 |
| SV (1/hr.) | 2980 | 2990 | 2990 | 1990 |
| WHSV (1/hr.) | 2130 | 2140 | 2140 | 1420 |
| O₂ conv., mole % | 66.728 | 62.627 | 98.304 | 99.496 |
| CH₄ conv. mole %(1) | 36.25 | 35.54 | 48.29 | 46.58 |
| CH₄ conv. mole %(2) | 35.94 | 34.37 | 47.58 | 45.88 |
| Res. time (sec.) | 0.123 | 0.123 | 0.117 | 0.176 |

| SELECTIVITIES, mole % | | | | |
|---|---|---|---|---|
| CO | 18.43 | 19.36 | 13.02 | 7.29 |
| CO₂ | 20.96 | 18.70 | 28.80 | 35.74 |
| C₂H₄ | 44.44 | 45.00 | 42.98 | 41.73 |
| C₂H₆ | 10.63 | 11.90 | 4.91 | 4.76 |
| C₂H₂ | 0.55 | 0.37 | 2.01 | 1.88 |
| C₃H₈ | 0.32 | 0.38 | 0.00 | 0.00 |
| C₃H₆ | 3.10 | 3.15 | 3.48 | 3.50 |
| Methyl acetylene | 0.18 | 0.13 | 0.71 | 0.81 |
| ALLENE | 0.00 | 0.00 | 0.34 | 0.36 |
| C₄'s | 0.00 | 1.01 | 2.40 | 2.33 |
| C₂₊ | 60.27 | 61.12 | 55.41 | 53.80 |
| C₅₊ | 0.16 | 0.00 | 1.34 | 1.61 |
| | | | | |
| Olefin ratio | 4.47 | 3.96 | 10.09 | 10.15 |
| C₂H₄/C₂H₆ | 4.18 | 3.78 | 8.76 | 8.76 |
| H₂/CO | 0.00 | 0.00 | 0.00 | 0.00 |
| CO/CO₂ | 0.88 | 1.04 | 0.45 | 0.20 |

| | | | | |
|---|---|---|---|---|
| (1) CH₄ conversion calculated from CH₄ in minus CH₄ out. | | | | |
| (2) CH₄ conversion calculated from carbon in products. | | | | |

**TABLE 12**

| REACTION CONDITIONS | | | | |
|---|---|---|---|---|
| Hours into run | 12:17 | 13:59 | 15:40 | 17:21 |
| Temp. C (Avg.) | 800.1 | 800.0 | 844.0 | 849.3 |
| Flow Rate (ml/min.) | 301.5 | 152.3 | 301.5 | 152.3 |
| SV (1/hr.) | 4520 | 2290 | 4520 | 2290 |
| WHSV (1/hr.) | 4070 | 2050 | 4070 | 2050 |
| O₂ conv., mole % | 30.397 | 72.404 | 78.543 | 98.566 |
| CH₄ conv. mole %(1) | 11.57 | 20.08 | 21.88 | 23.49 |
| CH₄ conv. mole %(2) | 11.51 | 20.01 | 21.76 | 23.38 |
| Res. time (sec.) | 0.081 | 0.160 | 0.078 | 0.153 |

| SELECTIVITIES, mole % | | | | |
|---|---|---|---|---|
| CO | 7.51 | 7.98 | 7.82 | 3.58 |
| CO₂ | 11.38 | 21.11 | 20.92 | 29.20 |
| C₂H₄ | 36.55 | 44.38 | 45.53 | 45.37 |
| C₂H₆ | 40.82 | 21.02 | 19.69 | 12.57 |
| C₂H₂ | 0.00 | 0.22 | 0.52 | 1.24 |
| C₃H₈ | 0.95 | 0.52 | 0.36 | 0.00 |
| C₃H₆ | 2.44 | 3.75 | 3.87 | 4.73 |
| Methyl acetylene | 0.00 | 0.12 | 0.26 | 0.74 |
| ALLENE | 0.00 | 0.06 | 0.14 | 0.33 |
| C₄'s | 0.00 | 0.85 | 0.89 | 1.69 |
| C₂₊ | 81.11 | 70.21 | 70.25 | 65.60 |
| C₅₊ | 0.00 | 0.00 | 0.00 | 0.55 |
| | | | | |
| Olefin ratio | 0.94 | 2.26 | 2.50 | 4.13 |
| C₂H₄/C₂H₆ | 0.90 | 2.11 | 2.31 | 3.61 |
| H₂CO | 0.00 | 0.00 | 0.00 | 0.00 |
| CO/CO₂ | 0.66 | 0.38 | 0.37 | 0.12 |

| | | | | |
|---|---|---|---|---|
| (1) CH₄ conversion calculated from CH₄ in minus CH₄ out. | | | | |
| (2) CH₄ conversion calculated from carbon in products. | | | | |

**TABLE 13**

| REACTION CONDITIONS | | | | |
|---|---|---|---|---|
| Hours into run | 11:54 | 13:35 | 15:16 | 16:58 |
| Temp. C (Avg.) | 800.0 | 799.8 | 847.7 | 849.0 |
| Flow Rate (ml/min) | 299.8 | 150.1 | 299.8 | 149.9 |
| SV (1/hr.) | 4500 | 2250 | 4500 | 2250 |
| WHSV (1/hr.) | 4120 | 2060 | 4120 | 2060 |
| O₂ conv., mole % | 45.523 | 68.970 | 71.150 | 88.345 |
| CH₄ conv. mole %(1) | 14.15 | 18.31 | 19.68 | 21.81 |
| CH₄ conv. mole %(2) | 14.66 | 18.58 | 19.86 | 21.85 |
| Res. time (sec.) | 0.082 | 0.163 | 0.078 | 0.156 |

| SELECTIVITIES, mole % | | | | |
|---|---|---|---|---|
| CO | 19.98 | 24.83 | 22.12 | 20.78 |
| CO₂ | 10.52 | 14.53 | 12.63 | 17.70 |
| C₂H₄ | 51.09 | 48.16 | 51.03 | 46.50 |
| C₂H₆ | 13.73 | 6.63 | 7.15 | 4.48 |
| C₂H₂ | 0.00 | 0.78 | 0.88 | 1.77 |
| C₃H₈ | 0.00 | 0.00 | 0.00 | 0.00 |
| C₃H₆ | 1.67 | 1.60 | 2.19 | 2.83 |
| Methyl acetylene | 0.00 | 0.08 | 0.21 | 0.47 |
| ALLENE | 0.00 | 0.06 | 0.09 | 0.21 |
| C4's | 2.50 | 2.05 | 2.34 | 2.31 |
| C₂₊ | 68.98 | 59.21 | 63.59 | 57.89 |
| C₅₊ | 0.52 | 1.35 | 1.35 | 2.95 |
| | | | | |
| Olefin ratio | 4.03 | 7.82 | 7.64 | 10.88 |
| C₂H₄/C₂H₆ | 3.72 | 7.26 | 7.14 | 10.39 |
| H₂/CO | 1.90 | 1.71 | 1.75 | 1.17 |
| CO/CO₂ | 1.90 | 1.71 | 1.75 | 1.17 |

| | | | | |
|---|---|---|---|---|
| (1) CH₄ conversion calculated from CH₄ in minus CH₄ out. | | | | |
| (2) CH₄ conversion calculated from carbon in products. | | | | |

### Discussion of Results:

Relative feed rates varied with the activity of the contact material but fell in the range of 2,000 to 50,000 standard cc per hour per cc. contact material. The activity and selectivity for C₂₊ hydrocarbons of contact materials of Examples 1-5 at various flow rates and temperatures can be seen in Tables 1-5. Contact materials that had higher amounts of alumina diluent had lower C₂₊ selectivities since the alumina's acidity contributes to the combustion activity. With these less active contact materials the activity due to the alumina becomes more significant. The best C₂₊ yields of 25%-27% came from the YBa₂GeOy(Cl) run with little or no alumina dilution and a feed ratio of CH₄/O₂ = 2:1. Olefin/paraffin ratios are in the range of 5-10 for the Y/Ba/Ge samples and were lower when the Ge was replaced with Ga.

### Examples 37-40

Contact material of Example 27 was examined in a series of experiments. The contact material screened to 40/60 mesh and 0.25 gm was mixed with 1.5 gms of 40/60 mesh Vycor glass. The mix was loaded in a 8mm (inside diameter) quartz tube with a 3mm (o.d.) quartz thermowell along the center line of the tube. The bed length was about 3.5 cm. Space above and below the bed was filled with 14/20 mesh quartz.

The reactor tube was placed in a 15 cm single-zone furnace. An external gas recycle pump was connected to the end couplings. Feed gases were preblended and compositions given are vendor analyses (Matheson). Ultrapure gases were used in all experiments.

### Example 37

Replicate, on-line, gas chromatographic analyses of the feed gas were obtained during the first 4 hours. A nominal 2:1 CH₄/O₂ feed gas blend was used. Analyzed values were 14.72% CH₄, 7.47% O₂, balance N₂. The reactor was heated rapidly to temperature and GC samples taken about every hour. Water was adsorbed on calcium sulfate prior to analysis. GC#'s and Temperatures are as follows:

| GC | Temp. °(C) | Fresh Feed Rate (sccm) |
|---|---|---|
| 5-9 | 750 | 100 |
| 10-12 | 700 | 100 |
| 13-15 | 650 | 100 |
| 16-18 | 600 | 100 |
| 19-20 | 750 | 100 |

### Discussion of Results:

Conversions, even at 750°C, were very low. At such low conversions, very little product is made and the relative uncertainties, just due to uncertainties in measurements, are large. At 750°C, methane conversion was about 3% and the selectivity to C₂₊ hydrocarbons was about 75%. Selectivity to CO and CO₂ were about equal at 12%-13% each.

### Example 38

Due to the low conversion of methane realized in Example 37, the feed rate was reduced to 10 sccm -- 10% of that used in Example 37. A gas recycle rate of about 90 sccm was also used to reduce thermal reactions which might begin at such a low fresh feed rate. The same contact material bed was kept from the previous example.

### Discussion of Results:

At 750°C, the C₂₊ selectivity was 48% with methane conversion of about 23%. Selectivity to CO was about 18% and to CO₂ was about 35%.

### Example 39

Testing was continued with the same loading of contact material as in Example 38. Conditions were fixed at 750°C, fresh feed of 5 sccm, and recycle gas at about 100 ccm. Eight GC samples were taken over about 8 hours.

### Discussion of Results:

These results showed a slightly higher conversion of methane due to the lower feed rate but essentially the same selectivities for C₂₊ hydrocarbons as the 750°C conditions of Example 38.

### Example 40

The same loading of contact material as used in Example 39 was used but a different feed gas feed was used. Analyzed composition of the new feed gas was 23.87% CH₄, 5.13% O₂, balance N₂ -- a nominal 5:1 methane to oxygen ratio. Cool product gas recycle was kept at 100 ccm and the feed rate and temperature was varied as follows:

| GG | Temp. °(C) | Fresh Feed Rate (sccm) |
|---|---|---|
| 1-4 | 750 | 5 |
| 5-7 | 800 | 5 |
| 8-15 | 800 | 10 |

At the end of this run, the contact material was unloaded. The used contact material was white and free flowing. The fresh contact material was also white. Thus the contact material did not coke as determined by visual inspection.

### Example 41

### ACTIVITY MAINTENANCE

The contact material of Example 29 was sieved to 40-60 mesh and a 2.0 gm portion was loaded in a quartz tube. The loaded quartz tube was placed in a three zone electric tube furnace.

The material was tested, at 850°C, for the oxidative conversion of methane to higher hydrocarbons for a period of time on stream of 1300 hours. In this testing, a feed gas blend of 40% CH_{4,} 4% O₂, and balance N₂ was passed over the contact material sample at a feed rate of 150 standard cubic centimeters per minute (sccm).

### Discussion of Results:

Both methane conversion and C₂₊ selectivity were fairly constant over the entire test time on stream. These results show a high degree of activity maintenance for the tested material.

The foregoing detailed description is given for clearness of understanding only, and no unnecessary limitations are to be understood therefrom, as modifications within the scope of the invention will be obvious to those skilled in the art.

## Claims

1. A method for converting lower alkanes to a product composition comprising a higher molecular weight hydrocarbon, comprising contacting a feed composition comprising at least one lower alkane material with an oxidative coupling contact material consisting of an intimately mixed, mixed oxide of:
a) at least one cationic species of a naturally occurring Group IIIB element selected from the group consisting of yttrium, lanthanum, neodymium, samarium and ytterbium;
b) at least one cationic species of a Group IIA metal selected from the group consisting of magnesium, calcium, strontium and barium, where the Group IIIB element cationic species and the Group IIA metal cationic species are present in an approximate molar ratio of about 1 Group IIIB element cationic species to about 0.5 to about 3 of the Group IIA metal cationic species; and
c) at least one additional metal cationic species selected from zirconium, hafnium, germanium, gallium and aluminum and wherein:
i) when selected from zirconium and hafnium, said additional metal cationic species is present in an amount in a range of about 10 to 55 weight percent of the oxidative coupling contact material;
ii) when selected from germanium and gallium, said additional metal cationic species is present in a molar ratio of about 1 mole of Group IIIB element cationic species to about 0.5 - 4 moles of the additional metal cationic species, and wherein the oxidative coupling contact material contains a halide, present in an amount in a range of about 5 to 20 weight percent of the oxidative coupling contact material; and
iii) when selected from aluminum, said additional metal cationic species is present in an amount in a range of about 2 to 20 weight percent of the oxidative coupling contact material;
with the contacting being at oxidative coupling reaction conditions and in the presence of oxygen.

2. The method of Claim 1 wherein the additional metal cationic species is zirconium or hafnium.

3. The method of Claim 1 wherein the additional metal cationic species is germanium or gallium.

4. The method of Claim 3 wherein the halide is chlorine.

5. The method of Claim 1 wherein the additional metal cationic species is aluminum.

6. The method of any preceding claim wherein the Group IIIB element is yttrium.

7. The method of any preceding claim wherein the Group IIA metal is strontium or barium.

8. The method of any preceding claim wherein methane is converted to a product composition comprising a higher molecular weight hydrocarbon.

9. A method according to Claim 1 for converting methane to a product composition comprising a higher molecular weight hydrocarbon, comprising contacting a feed composition comprising methane with an oxidative coupling contact material consisting of an intimately mixed halogen-containing mixed oxide of:
a) at least one cationic species of a naturally occurring Group IIIB element selected from the group consisting of yttrium, lanthanum, neodymium, samarium and ytterbium;
b) at least one cationic species of a Group IIA metal selected from the group consisting of magnesium, calcium, strontium and barium, where the Group IIIB element cationic species and the Group IIA metal cationic species are present in an approximate molar ratio of about 1 Group IIIB element cationic species to about 0.5 to about 3 of the Group IIA metal cationic species, wherein the oxidative coupling contact material contains a halide, present in an amount in a range of about 5 to 20 weight percent of the oxidative coupling contact material; and
c) at least one additional metal cationic species selected from germanium and gallium, present in a molar ratio of about 1 mole of Group IIIB element cationic species to about 0.5 - 4 moles of the additional metal cationic species;
with the contacting being at oxidative coupling reaction conditions and in the presence of oxygen.

10. The method of Claim 9 wherein the Group IIIB element is yttrium.

11. The method of Claim 9 or Claim 10 wherein the Group IIA metal is barium.

12. The method of any of Claims 9 to 11 wherein the oxidative coupling contact material comprises the halogen chlorine, present in an amount in a range of about 8 to 17 weight percent of the oxidative coupling contact material.

13. The method of any of Claims 9 to 12 wherein contact material can be represented by the chemical formula:
Y(BaCl₂)₂GeO₃₅

14. An oxidative coupling contact material consisting of an intimately mixed, mixed oxide of:
a) at least one cationic species of a Group IIIB element selected from the group consisting of yttrium, lanthanum, neodymium, samarium and ytterbium;
b) at least one cationic species of a Group IIA metal selected from the group consisting of strontium and barium, where the Group IIIB element cationic species and the Group IIA metal cationic species are present in an approximate molar ratio of about 1 Group IIIB element cationic species to about 0.5 to about 3 of the Group IIA metal cationic species; and
c) at least one additional metal cationic species selected from zirconium, hafnium, germanium, gallium and aluminum and wherein:
i) when selected from zirconium and hafnium, said additional metal cationic species is present in an amount in a range of about 10 to 55 weight percent of the oxidative coupling contact material;
ii) when selected from germanium and gallium, said additional metal cationic species is present in a molar ratio of about 1 mole of Group IIIB element cationic species to about 0.5 - 4 moles of the additional metal cationic species, and wherein the oxidative coupling contact material contains a halide, present in an amount in a range of about 5 to 20 weight percent of the oxidative coupling contact material; and
iii) when selected from aluminum, said additional metal cationic species ispresent in an amount in a range of about 2 to 20 weight percent of the oxidative coupling contact material.

15. The oxidative coupling contact material of Claim 14 wherein the additional metal cationic species is zirconium or hafnium.

16. The oxidative coupling contact material of Claim 14 wherein the additional metal cationic species is germanium or gallium.

17. The contact material of Claim 16 wherein the halide is chlorine.

18. The oxidative coupling contact material of Claim 14 wherein the additional metal cationic species is aluminum.

19. The oxidative coupling contact material of any of Claims 14 to 18 wherein the Group IIIB element is yttrium.

20. The oxidative coupling contact material of any of Claims 14 to 18 wherein the Group IIA metal is strontium or barium.

## Patentansprüche

1. Verfahren zum Umwandeln von niederen Alkanen zu einer Produktzusammensetzung, welche einen Kohlenwasserstoff mit höherem Molekulargewicht umfaßt, welches Verfahren das In-Kontakt-Bringen einer Ausgangszusammensetzung, welche mindestens ein niederes Alkanmaterial umfaßt, mit einem Kontaktmaterial für eine oxidative Kupplung umfaßt, welches aus einem innig durchmischten Mischoxid aus:
a) mindestens einer kationischen Spezies eines natürlich vorkommenden Elements der Gruppe IIIB, ausgewählt aus der Gruppe, bestehend aus Yttrium, Lanthan, Neodym, Samarium und Ytterbium;
b) mindestens einer kationischen Spezies eines Metalls der Gruppe IIA, ausgewählt aus der Gruppe, bestehend aus Magnesium, Calcium, Strontium und Barium, wobei die kationische Spezies des Elements der Gruppe IIIB und die kationische Spezies des Metalls der Gruppe IIA in einem ungefähren Molverhältnis von ungefähr 1 kationischen Spezies des Elements der Gruppe IIIB zu ungefähr 0,5 bis ungefähr 3 kationischen Spezies des Metalls der Gruppe IIA vorhanden sind; und
c) mindestens einer zusätzlichen kationischen Metallspezies, ausgewählt aus Zirkonium, Hafnium, Germanium, Gallium und Aluminium, und wobei:
i) wenn aus Zirkonium und Hafnium ausgewählt wird, die zusätzliche kationische Metallspezies in einer Menge im Bereich von ungefähr 10 bis 55 Gew.-% des Kontaktmaterials für eine oxidative Kupplung vorhanden ist;
ii) wenn aus Germanium und Gallium ausgewählt wird, die zusätzliche kationische Metallspezies in einem Molverhältnis von ungefähr 1 mol der kationischen Spezies des Elements der Gruppe IIIB zu ungefähr 0,5 bis 4 mol der zusätzlichen kationischen Metallspezies vorhanden ist, und wobei das Kontaktmaterial für eine oxidative Kupplung ein Halogenid enthält, welches in einer Menge im Bereich von ungefähr 5 bis 20 Gew.-% des Kontaktmaterials für eine oxidative Kupplung vorhanden ist; und
iii) wenn aus Aluminium ausgewählt wird, die zusätzliche kationische Metallspezies in einer Menge im Bereich von ungefähr 2 bis 20 Gew.-% des Kontaktmaterials für eine oxidative Kupplung vorhanden ist;
besteht, wobei das In-Kontakt-Bringen bei Umsetzungsbedingungen einer oxidativen Kupplung und in Gegenwart von Sauerstoff geschieht.

2. Verfahren gemäß Anspruch 1, wobei die zusätzliche kationische Metallspezies Zirkonium oder Hafnium ist.

3. Verfahren gemäß Anspruch 1, wobei die zusätzliche kationische Metallspezies Germanium oder Gallium ist.

4. Verfahren gemäß Anspruch 3, wobei das Halogenid Chlor ist.

5. Verfahren gemäß Anspruch 1, wobei die zusätzliche kationische Metallspezies Aluminium ist.

6. Verfahren gemäß einem der vorhergehenden Ansprüche, wobei das Element der Gruppe IIIB Yttrium ist.

7. Verfahren gemäß einem der vorhergehenden Ansprüche, wobei das Metall der Gruppe IIA Strontium oder Barium ist.

8. Verfahren gemäß einem der vorhergehenden Ansprüche, wobei Methan zu einer Produktzusammensetzung umgewandelt wird, welche einen Kohlenwasserstoff mit höherem Molekulargewicht umfaßt.

9. Verfahren gemäß Anspruch 1, zum Umwandeln von Methan zu einer Produktzusammensetzung, welche einen Kohlenwasserstoff mit höherem Molekulargewicht umfaßt, welches Verfahren das In-Kontakt-Bringen einer Methan umfassenden Ausgangszusammensetzung mit einem Kontaktmaterial für eine oxidative Kupplung umfaßt, welches aus einem innig durchmischten halogenhaltigen Mischoxid aus:
a) mindestens einer kationischen Spezies eines natürlich vorkommenden Elements der Gruppe IIIB, ausgewählt aus der Gruppe, bestehend aus Yttrium, Lanthan, Neodym, Samarium und Ytterbium;
b) mindestens einer kationischen Spezies eines Metalls der Gruppe IIA, ausgewählt aus der Gruppe, bestehend aus Magnesium, Calcium, Strontium und Barium, wobei die kationische Spezies des Elements der Gruppe IIIB und die kationische Spezies des Metalls der Gruppe IIA in einem ungefähren Molverhältnis von ungefähr 1 kationischen Spezies des Elements der Gruppe IIIB zu ungefähr 0,5 bis ungefähr 3 kationischen Spezies des Metalls der Gruppe IIA vorhanden sind, wobei das Kontaktmaterial für eine oxidative Kupplung ein Halogenid enthält, welches in einer Menge im Bereich von ungefähr 5 bis 20 Gew.-% des Kontaktmaterials für eine oxidative Kupplung vorhanden ist; und
c) mindestens einer zusätzlichen kationischen Metallspezies, ausgewählt aus Germanium und Gallium, welche in einem Molverhältnis von ungefähr 1 mol der kationischen Spezies des Elements der Gruppe IIIB zu ungefähr 0,5 bis 4 mol der zusätzlichen kationischen Metallspezies vorhanden ist;
besteht, wobei das In-Kontakt-Bringen bei Umsetzungsbedingungen einer oxidativen Kupplung und in Gegenwart von Sauerstoff geschieht.

10. Verfahren gemäß Anspruch 9, wobei das Element der Gruppe IIIB Yttrium ist.

11. Verfahren gemäß Anspruch 9 oder Anspruch 10, wobei das Metall der Gruppe IIA Barium ist.

12. Verfahren gemäß einem der Ansprüche 9 bis 11, wobei das Kontaktmaterial für eine oxidative Kupplung, welches das Halogenid Chlor umfaßt, welches in einer Menge im Bereich von ungefähr 8 bis 17 Gew.-% des Kontaktmaterials für eine oxidative Kupplung vorhanden ist.

13. Verfahren gemäß einem der Ansprüche 9 bis 12, wobei das Kontaktmaterial durch die chemische Formel:
Y(BaCl₂)₂GeO_{3,5}
ausgedrückt werden kann.

14. Kontaktmaterial für eine oxidative Kupplung, welches aus einem innig durchmischten Mischoxid aus:
a) mindestens einer kationischen Spezies eines Elements der Gruppe IIIB, ausgewählt aus der Gruppe, bestehend aus Yttrium, Lanthan, Neodym, Samarium und Ytterbium;
b) mindestens einer kationischen Spezies eines Metalls der Gruppe IIA, ausgewählt aus der Gruppe, bestehend aus Strontium und Barium, wobei die kationische Spezies des Elements der Gruppe IIIB und die kationische Spezies des Metalls der Gruppe IIA in einem ungefähren Molverhältnis von ungefähr 1 kationischen Spezies des Elements der Gruppe IIIB zu ungefähr 0,5 bis ungefähr 3 kationischen Spezies des Metalls der Gruppe IIA vorhanden sind; und
c) mindestens einer zusätzlichen kationischen Metallspezies, ausgewählt aus Zirkonium, Hafnium, Germanium, Gallium und Aluminium, und wobei:
i) wenn aus Zirkonium und Hafnium ausgewählt wird, die zusätzliche kationische Metallspezies in einer Menge im Bereich von ungefähr 10 bis 55 Gew.-% des Kontaktmaterials für eine oxidative Kupplung vorhanden ist;
ii) wenn aus Germanium und Gallium ausgewählt wird, die zusätzliche kationische Metallspezies in einem Molverhältnis von ungefähr 1 mol der kationischen Spezies des Elements der Gruppe IIIB zu ungefähr 0,5 bis 4 mol der zusätzlichen kationischen Metallspezies vorhanden ist, und wobei das Kontaktmaterial für eine oxidative Kupplung ein Halogenid enthält, welches in einer Menge im Bereich von ungefähr 5 bis 20 Gew.-% des Kontaktmaterials für eine oxidative Kupplung vorhanden ist; und
iii) wenn aus Aluminium ausgewählt wird, die zusätzliche kationische Metallspezies in einer Menge im Bereich von ungefähr 2 bis 20 Gew.-% des Kontaktmaterials für eine oxidative Kupplung vorhanden ist;
besteht.

15. Kontaktmaterial für eine oxidative Kupplung gemäß Anspruch 14, wobei die zusätzliche kationische Metallspezies Zirkonium oder Hafnium ist.

16. Kontaktmaterial für eine oxidative Kupplung gemäß Anspruch 14, wobei die zusätzliche kationische Metallspezies Germanium oder Gallium ist.

17. Kontaktmaterial für eine oxidative Kupplung gemäß Anspruch 16, wobei das Halogenid Chlor ist.

18. Kontaktmaterial für eine oxidative Kupplung gemäß Anspruch 14, wobei die zusätzliche kationische Metallspezies Aluminium ist.

19. Kontaktmaterial für eine oxidative Kupplung gemäß einem der Ansprüche 14 bis 18, wobei das Element der Gruppe IIIB Yttrium ist.

20. Kontaktmaterial für eine oxidative Kupplung gemäß einem der Ansprüche 14 bis 18, wobei das Metall der Gruppe IIA Strontium oder Barium ist.

## Revendications

1. Procédé de conversion d'alcanes inférieurs en une composition de produits contenant un hydrocarbure à poids moléculaire élevé, comprenant la mise en contact d'une composition d'alimentation contenant au moins un matériau alcane inférieur, avec un matériau de contact par couplage oxydant consistant en un mélange intime d'oxyde mixte
(a) d'au moins une espèce cationique d'un élément du Groupe IIIB existant dans la nature, choisi au sein de l'ensemble comprenant l'yttrium, le lanthane, le néodyme, le samarium et l'ytterbium ;
(b) d'au moins une espèce cationique d'un métal du Groupe IIA choisi au sein de l'ensemble comprenant le magnésium, le calcium, le strontium, et le baryum, où l'espèce cationique de l'élément du Groupe IIIB et l'espèce cationique du métal du Groupe IIA sont présentes dans un ratio molaire approximatif correspondant à environ 1 pour l'espèce cationique d'élément de Groupe IIIB pour environ 0,5 à environ 3 pour l'espèce cationique du métal du Groupe IIA ; et
(c) d'au moins une espèce cationique supplémentaire d'un métal choisi parmi le zirconium, l'hafnium, le germanium, le gallium et l'aluminium , et dans lequel
- (i) lorsqu'elle est choisie parmi le zirconium et l'hafnium, ladite espèce métallique cationique supplémentaire est présente en quantité comprise entre environ 10 à 55 pour cent en poids, du matériau de contact par couplage oxydant ;
- (ii) lorsqu'elle est choisie parmi le germanium et le gallium, ladite espèce métallique cationique supplémentaire est présente dans un ratio molaire d'environ 1 mole d'espèce cationique de l'élément du Groupe IIIB pour environ 0,5 à 4 moles de l'espèce métallique cationique supplémentaire, et dans lequel le matériau de contact par couplage oxydant contient un halogénure présent en quantité comprise entre 5 et 20 pour cent en poids, du matériau de contact par couplage oxydant ; et
- (iii) lorsque le choix se porte sur l'aluminium, ladite espèce cationique métallique supplémentaire est présente en quantité comprise entre environ 2 et 20 pour cent en poids, du matériau de contact par couplage oxydant ;
la mise en contact se faisant dans des conditions de réaction de couplage oxydant et en présence d'oxygène.

2. Procédé selon la revendication 1, dans lequel l'espèce cationique métallique supplémentaire est le zirconium ou l'hafnium.

3. Procédé selon la revendication 1, dans lequel l'espèce cationique métallique supplémentaire est le germanium ou le gallium.

4. Procédé selon la revendication 3, dans lequel l'halogénure est à base de chlore.

5. Procédé selon la revendication 1, dans lequel l'espèce cationique métallique supplémentaire est l'aluminium.

6. Procédé selon l'une quelconque des revendications précédentes, dans lequel l'élément du Groupe IIIB est l'yttrium.

7. Procédé selon l'une quelconque des revendications précédentes, dans lequel le métal du Groupe IIA est le strontium ou le baryum.

8. Procédé selon l'une quelconque des revendications précédentes, dans lequel le méthane est converti en une composition de produits contenant un hydrocarbure de poids moléculaire élevé.

9. Procédé selon la revendication 1, pour la conversion du méthane en une composition de produits contenant un hydrocarbure à poids moléculaire élevé, comprenant la mise en contact d'une composition d'alimentation contenant du méthane, avec un matériau de contact par couplage oxydant consistant en un mélange intime d'oxyde mixte :
(a) d'au moins une espèce cationique d'un élément du Groupe IIIB existant dans la nature, choisi au sein de l'ensemble comprenant l'yttrium, le lanthane, le néodyme, le samarium et l'ytterbium ;
(b) d'au moins une espèce cationique d'un métal du Groupe IIA choisi au sein de l'ensemble comprenant le magnésium, le calcium, le strontium, et le baryum, où l'espèce cationique de l'élément du Groupe IIIB et l'espèce cationique du métal du Groupe IIA sont présentes dans un ratio molaire approximatif correspondant à environ 1 pour l'espèce cationique de l'élément du Groupe IIIB pour environ 0,5 à environ 3 pour l'espèce cationique du métal du Groupe IIA ; et dans lequel le matériau de contact par couplage oxydant contient un halogénure présent en quantité d'environ 5 à 20 pour cent en poids de matériau de contact par couplage oxydant et
(c) d'au moins une espèce métallique cationique supplémentaire choisie parmi le germanium et le gallium, présente dans un ratio moléculaire correspondant à environ 1 mole d'espèce cationique de l'élément du Groupe IIIB pour environ 0,5 à moles de l'espèce cationique métallique supplémentaire ;
la mise en contact se faisant dans des conditions de réaction de couplage oxydant et en présence d'oxygène.

10. Procédé selon la revendication 9, dans lequel l'élément du Groupe IIIB est l'yttrium.

11. Procédé selon la revendication 9 ou la revendication 10 dans lequel le métal du Groupe IIA est le baryum.

12. Procédé selon l'une quelconque des revendications 9 à 11, dans lequel le matériau de contact par couplage oxydant contient l'halogène chlore, présent en quantité comprise entre environ 8 et 17 pour cent en poids du matériau de contact par couplage oxydant.

13. Procédé selon l'une quelconque des revendications 9 à 12 dans lequel le matériau de contact peut être représenté par la formule :
Y(BaCl₂)₂GeO_{3,5}

14. Matériau de contact par couplage oxydant consistant en un mélange intime d'oxyde mixte
(a) d'au moins une espèce cationique d'un élément du Groupe IIIB, choisi au sein de l'ensemble comprenant l'yttrium, le lanthane, le néodyme, le samarium et l'ytterbium ;
(b) d'au moins une espèce cationique d'un métal du Groupe IIA choisi au sein de l'ensemble comprenant le strontium et le baryum, où l'espèce cationique de l'élément du Groupe IIIB et l'espèce cationique du métal du Groupe lIA sont présentes dans un ratio molaire approximatif correspondant à environ 1 pour l'espèce cationique de l'élément du Groupe IIIB pour environ 0,5 à environ 3 pour l'espèce cationique du métal du Groupe IIA; et
(c) d'au moins une espèce cationique supplémentaire d'un métal choisi parmi le zirconium, l'hafnium, le germanium, le gallium et l'aluminium, et dans lequel
- (i) lorsqu'elle est choisie parmi le zirconium et l'hafnium, ladite espèce métallique cationique supplémentaire est présente en quantité comprise entre environ 10 à 55 pour cent en poids, du matériau de contact par couplage oxydant ;
- (ii) lorsqu'elle est choisie parmi le germanium et le gallium, ladite espèce métallique cationique supplémentaire est présente dans un ratio molaire d'environ 1 mole d'espèce cationique de l'élément du Groupe IIIB pour environ 0,5 à 4 moles de l'espèce métallique cationique supplémentaire, et dans lequel le matériau de contact par couplage oxydant contient un halogénure présent en quantité comprise entre 5 et 20 pour cent en poids, du matériau de contact par couplage oxydant ; et
- (iii) lorsque le choix se porte sur l'aluminium, ladite espèce cationique métallique supplémentaire est présente en quantité comprise entre environ 2 et 20 pour cent en poids, du matériau de contact par couplage oxydant.

15. Matériau de contact par couplage oxydant selon la revendication 14, dans lequel l'espèce métallique cationique supplémentaire est le zirconium ou l'hafnium.

16. Matériau de contact par couplage oxydant selon la revendication 14, dans lequel l'espèce métallique cationique supplémentaire est le gennanium ou le gallium.

17. Matériau de contact selon la revendication 16, dans lequel l'halogénure est à base de chlore.

18. Matériau de contact par couplage oxydant selon la revendication 14, dans lequel l'espèce métallique cationique supplémentaire est l'aluminium.

19. Matériau de contact par couplage oxydant selon l'une quelconque des revendications 14 à 18, dans lequel l'élément du Groupe IIIB est l'yttrium.

20. Matériau de contact par couplage oxydant selon l'une quelconque des revendications 14 à 18; dans lequel le métal du Groupe IIA est le strontium ou le baryum.
